(19) Europäisches Patentamt
European Patent Office
Office européen des brevets

(11) **EP 4 275 597 B1**

(12) **FASCICULE DE BREVET EUROPEEN**

(45) Date de publication et mention de la délivrance du brevet:
**03.07.2024 Bulletin 2024/27**

(21) Numéro de dépôt: **22173132.6**

(22) Date de dépôt: **12.05.2022**

(51) Classification Internationale des Brevets (IPC):
**A61B 5/145** (2006.01)   **A61B 5/1455** (2006.01)
**A61B 5/00** (2006.01)   **G01N 21/27** (2006.01)
**G16H 50/20** (2018.01)

(52) Classification Coopérative des Brevets (CPC):
**A61B 5/0095; A61B 5/14532; A61B 5/1455;
A61B 5/7264; A61B 5/7267; G01N 21/1702;
G01N 21/171; G16H 40/63; G16H 50/20;**
A61B 5/0093; A61B 2560/0223; G01N 2021/1706;
G01N 2021/1731

(54) **CAPTEUR NON INVASIF ET PROCEDE DE MESURE**

NICHT-INVASIVER SENSOR UND MESSVERFAHREN

NON-INVASIVE SENSOR AND MEASURING METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**15.11.2023 Bulletin 2023/46**

(73) Titulaire: **Eclypia**
**38000 GRENOBLE (FR)**

(72) Inventeurs:
• **MONPEURT, Cyrielle**
**38000 GRENOBLE (FR)**
• **BLANC, Romain**
**38000 GRENOBLE (FR)**
• **GALLEGOS, Alexandre**
**75002 PARIS (FR)**

(74) Mandataire: **Fidal Innovation**
**4-6 avenue d'Alsace**
**92400 Courbevoie (FR)**

(56) Documents cités:
**EP-A1- 2 460 470       US-A1- 2003 023 152
US-A1- 2017 042 428**

• **TRONSTAD CHRISTIAN ET AL: "Non-invasive prediction of blood glucose trends during hypoglycemia", ANALYTICA CHIMICA ACTA, vol. 1052, 15 décembre 2018 (2018-12-15), pages 37-48, XP085583751, ISSN: 0003-2670, DOI: 10.1016/J.ACA.2018.12.009**

**Description**

**DOMAINE TECHNIQUE DE L'INVENTION**

La présente invention se rapporte à un procédé de mesure et à un capteur non invasif permettant de mesurer un ou plusieurs paramètres d'intérêt dans un milieu cible.

Plus précisément, l'invention se rapporte à un capteur non invasif basé sur la détection d'un effet photothermique ou photoacoustique, notamment configuré pour mesurer un ou plusieurs paramètres dans un milieu cible stratifié, dont la stratification peut être évolutive au cours du temps. Un paramètre mesuré peut par exemple être la glycémie dans la peau.

**ARRIERE-PLAN TECHNOLOGIQUE**

Dans le domaine des capteurs pour les organismes vivants, il est connu de réaliser des capteurs non invasifs basés sur la détection photoacoustique ou photothermique.

Une zone d'intérêt d'un milieu à analyser, appelée cible, est irradiée au moyen d'un faisceau laser de longueur d'onde et de fréquence de modulation choisies en fonction du paramètre d'intérêt à mesurer. Le faisceau laser est absorbé par la cible sur une longueur caractéristique qui dépend de la structuration de la cible. L'absorption d'énergie lumineuse entraîne un échauffement local de la cible. En réaction à cet échauffement, une onde thermique de fréquence égale à la fréquence de modulation du laser est générée dans la cible. Cette onde se propage dans la cible et notamment jusqu'à la surface extérieure de la cible.

L'onde thermique peut être directement détectée et analysée. On parle alors de photothermie. La détection photoacoustique exploite quant à elle le fait que l'onde thermique est associée à une onde de pression de fréquence identique à la fréquence de modulation.

Dans le cas de la détection photoacoustique indirecte, on détecte l'onde de pression générée dans le milieu extérieur fluide lorsque l'onde thermique générée dans la cible parvient, après propagation, à l'interface cible - milieu extérieur fluide.

L'effet photoacoustique a fait l'objet de nombreuses études théoriques. Allan Rosencwaig et Allen Gersho ont notamment développé un modèle théorique du signal photoacoustique. Ce modèle met en jeu les propriétés physico-chimiques de l'échantillon à analyser, parmi lesquelles la longueur de diffusion optique, l'épaisseur et la longueur de diffusion thermique de l'échantillon. (Rosencwaig, A. and Gersho, A. (1976), Theory of the photoacoustic effect with solids, Journal of Applied Physics, 47, 64).

Hu et al. ont quant à eux développé une théorie généralisée de l'effet photoacoustique dans un matériau stratifié. (Hu, H., Wang, X., & Xu, X. (1999). Generalized theory of the photoacoustic effect in a multilayer material. Journal of Applied Physics, 86, 3953-3958.)

La détection photoacoustique présente de nombreux avantages par rapport à d'autres techniques de détection parmi lesquels on peut citer l'aspect orthogonal de la transduction : le signal optique en entrée du milieu à analyser est converti en un signal acoustique qui est très spécifique du phénomène à observer et qui permet d'utiliser des capteurs peu coûteux et miniaturisés.

La difficulté de la détection photoacoustique ou photothermique provient entre autres:

- du nombre de paramètres influençant en général le signal détecté et,
- pour certains analytes d'intérêt présents en concentration faible dans le milieu à analyser, de la faible proportion du signal détecté spécifique à chacun de ces paramètres d'intérêt.

La détection photoacoustique ou photothermique nécessite non seulement un choix de longueur d'onde mais aussi un choix de la ou des fréquences de modulation du laser à utiliser. En effet, la longueur caractéristique de pénétration de l'onde lumineuse incidente dans la cible dépend de la fréquence de modulation du laser. Si la structure de la cible (par exemple la peau) change au fur et à mesure du temps, la (ou les) fréquence(s) de modulation à utiliser pour mesurer le paramètre d'intérêt (par exemple la glycémie interstitielle) avec une précision et une consommation contrôlées change(nt) elle(s) aussi au cours du temps.

De plus, dans un milieu cible stratifié, les réflexions au niveau des interfaces entre les différentes strates donnent lieu à partir d'une onde thermique dite « primaire » se propageant vers la surface du milieu cible à une multitude d'ondes , dites « secondaire », « tertiaire », etc., qui sont susceptibles d'interférer les unes avec les autres comme l'on montré de nombreux auteurs. Les interférences d'ondes thermiques générées par effet photothermique ont notamment été décrites dans les documents suivants :

- C. A. Bennett and R. R. Patty, "Thermal wave interferometry: a potential application of the photoacoustic effect," Appl. Opt. 21, 49-54 (1982) ;

- Andreas Mandelis, "Theory of photothermal-wave diffraction and interférence in condensed media," J. Opt. Soc. Am. A 6, 298-308 (1989) ;
- Andreas Mandelis and Kwan F. Leung, "Photothermal-wave diffraction and interférence in condensed media: experimental evidence in aluminum," J. Opt. Soc. Am. A 8, 186-200 (1991). Ces interférences sont susceptibles d'affecter la précision d'un capteur non invasif basé sur la détection photoacoustique ou photothermique. Dans un milieu cible stratifié dont la stratification est susceptible d'évoluer, les conditions d'interférences sont elles aussi susceptibles d'évoluer puisqu'elles dépendent de la structuration du milieu cible. En conséquence, les conditions d'interférence ne sont pas constantes dans le temps.

L'invention vise donc à améliorer la précision d'un capteur non invasif dans un milieu cible, notamment stratifié et/ou évolutif basé sur la détection photoacoustique ou photothermique en prenant en compte les phénomènes d'interférences tout en contrôlant la consommation énergétique de ce capteur ou encore à réduire la consommation énergétique de ce capteur tout en contrôlant sa précision.

## RESUME DE L'INVENTION

Ainsi, l'invention se rapporte à un procédé de mesure d'un paramètre d'intérêt dans un milieu stratifié cible au moyen d'un capteur non invasif basé sur la détection photoacoustique ou la détection photothermique. Le procédé de mesure comprend :

a) on fournit un capteur comprenant :

- une source de lumière,
- un dispositif de contrôle d'une pluralité de paramètres d'irradiation de la source de lumière, la pluralité de paramètres d'irradiation comprenant au moins une fréquence de modulation de l'intensité de la source de lumière,
- une cellule de détection configurée pour détecter un signal acoustique ou thermique (12),
- une mémoire dans laquelle est stocké un abaque de fréquences nodales comprenant :

* pour une pluralité de groupes de configurations modèles du milieu stratifié cible, comprenant chacun au moins deux configurations modèles du milieu stratifié cible ne différant les unes des autres que par le paramètre d'intérêt,
* et pour une pluralité de groupes de cas d'irradiation comprenant chacun au moins deux cas d'irradiation ne différant les uns des autres que par une fréquence de modulation d'une source de lumière, chaque cas d'irradiation comprenant un jeu de valeurs de paramètres d'irradiation, une pluralité de multiplets nodaux, chaque multiplet nodal comprenant des caractéristiques communes à tous les éléments d'un groupe donné de configurations modèles du milieu stratifié cible et une pluralité de fréquences de modulation nodales associées, pour lesquelles le signal acoustique ou thermique détecté par la cellule de détection en réponse à une irradiation par la source de lumière présente une corrélation inférieure à un seuil prédéterminé avec le paramètre d'intérêt ;

- et un module d'adaptation comprenant un processeur et échangeant des informations avec la cellule de détection et le dispositif de contrôle des paramètres d'irradiation de la source de lumière ;

b) le module d'adaptation choisit une configuration modèle du milieu stratifié cible (CMirrad) initiale en vue d'une irradiation ;
c) le processeur du module d'adaptation :

- détermine dans l'abaque de fréquences nodales une pluralité de fréquences de modulation nodales associées à un groupe de configurations modèles du milieu stratifié cible auquel appartient la configuration modèle du milieu stratifié choisie,
- puis détermine au moins une fréquence de modulation particulière sur la base de ladite pluralité de fréquences de modulation nodales,
- et détermine un cas d'irradiation particulier pour la configuration modèle du milieu stratifié cible choisie, le cas d'irradiation particulier comprenant l'au moins une fréquence de modulation particulière;

d) la source de lumière irradie le milieu stratifié cible suivant le jeu de paramètres d'irradiation dudit cas d'irradiation particulier ;

e) la cellule de détection détecte un signal acoustique ou thermique généré en réponse à l'irradiation ;

f) le processeur du module d'adaptation détermine le paramètre d'intérêt sur la base du signal acoustique ou thermique détecté.

Grâce à ces dispositions, l'irradiation est réalisée selon un cas d'irradiation comprenant une fréquence de modulation d'au moins une source de lumière particulière, particulière en ce sens qu'elle a été choisie pour tenir compte des fréquences nodales correspondant à la configuration modèle du milieu stratifié cible retenue pour l'irradiation. Notamment, la fréquence de modulation particulière peut être déterminée sur la base de deux fréquences nodales, de manière à ce que la fréquence de modulation particulière corresponde à un maximum de sensibilité du capteur (toutes choses étant égales par ailleurs) pour l'intervalle compris entre ces deux fréquences de modulation.

Ces dispositions permettent donc d'améliorer la sensibilité du capteur non invasif par rapport à un capteur de l'art antérieur ne comprenant pas cette étape d'adaptation du cas d'irradiation en tenant compte des phénomènes d'interférence.

Par ailleurs, il est possible de choisir pour l'irradiation un nombre limité de fréquences de modulation, par exemple en ne retenant que la fréquence de modulation particulière ou un petit nombre de fréquences de modulation comprenant cette fréquence de modulation particulière. Ces dispositions permettent donc de réduire la puissance d'irradiation en conservant une sensibilité identique à celle d'un capteur de l'art antérieur.

Selon un mode de réalisation du procédé de mesure,

- le processeur du module d'adaptation (14) implémente de plus un algorithme de modélisation inverse recevant en entrée un cas d'irradiation (Ij) et un signal acoustique ou thermique et fournissant en sortie une configuration modèle (CMk) du milieu stratifié cible et une valeur du paramètre d'intérêt,
- et l'étape f) comprend les étapes suivantes :

  f1) le processeur du module d'adaptation reçoit en entrée le signal acoustique ou thermique détecté par la cellule de détection (12) et le cas d'irradiation particulier utilisé pour l'irradiation, et renvoie en sortie de l'algorithme de modélisation inverse une configuration modèle du milieu stratifié cible en cours (CMmes) et une valeur du paramètre d'intérêt estimée (Pest) ;

  f2) le processeur du module d'adaptation (14) évalue la configuration modèle du milieu stratifié cible choisie (CMirrad) par comparaison avec la configuration modèle du milieu stratifié cible en cours (CMmes), et seulement si cette évaluation est défavorable : g) le module d'adaptation (14) reçoit en entrée la configuration modèle du milieu stratifié cible en cours (CMmes) et renvoie en sortie une nouvelle configuration modèle du milieu stratifié cible choisie en vue d'une irradiation (CMirrad), puis on réitère c), d), e) et f1) ;

  f3) la valeur du paramètre d'intérêt mesurée (Pmes) par le capteur est la dernière valeur du paramètre d'intérêt estimée (Pest).

Grâce à ces dispositions, il est possible d'obtenir une mesure de glycémie avec :

- soit une seule irradiation (pas d'exécution de la sous-étape g, dans le cas d'une évaluation favorable). Cette irradiation est alors effectuée selon un cas d'irradiation choisi par défaut mais correspondant à une consommation énergétique contrôlée (les paramètres d'irradiation étant par exemple choisis en nombre limité). L'étape f2) d'évaluation de la configuration modèle d'irradiation permet d'acquérir la certitude que la précision de la mesure était bien la plus élevée possible pour le cas d'espèce au vu des cas d'irradiations disponibles dans la table de correspondance, ce qui est donc un avantage par rapport à un procédé dans lequel l'étape g) n'est pas prévue et pour lequel on ne dispose pas de cette certitude ;
- soit deux irradiations (étape f2) avec exécution de la sous-étape g), dans le cas d'une évaluation défavorable) et donc comprenant une réitération de c), d), e) et f1)). La seconde irradiation est alors effectuée selon le cas d'irradiation permettant d'obtenir la précision la plus élevée possible pour le cas d'espèce au vu des informations supplémentaires acquises grâce à la première irradiation et des cas d'irradiation disponibles dans la table de correspondance. La précision du procédé est donc connue et améliorée par rapport à un procédé ne présentant pas l'étape g). De plus, non seulement le cas d'irradiation choisi tient compte des phénomènes d'interférences pour la configuration modèle choisie pour la première irradiation mais encore le caractère optimal de la configuration modèle par rapport à l'état actuel du milieu stratifié est vérifiée, et si nécessaire une autre configuration modèle et un autre cas d'irradiation tenant compte des phénomènes d'interférences sont choisies pour une deuxième irradiation sur la base de laquelle le paramètre d'intérêt sera déterminé.

La précision et/ou la sensibilité et/ou la consommation énergétique du capteur non invasif peuvent ainsi encore être améliorés en continu, au fur et à mesure des évolutions du milieu stratifié cible.

Selon un mode de réalisation du procédé de mesure, g1) comprend en outre on réitère f2) à l'issue de f1).

Grâce à cette disposition, le choix de la configuration modèle et du cas d'irradiation tenant compte des phénomènes d'interférences sont réitérés jusqu'à atteindre les performances souhaitées en termes de précision et/ou de sensibilité et/ou de consommation énergétique du capteur non invasif. On ne se limite donc pas à une ou deux irradiations successives, trois irradiations ou plus pouvant au final permettre d'atteindre une meilleure sensibilité pour la situation du milieu stratifié cible à l'instant de la mesure qu'avec une ou deux irradiations, ou bien un meilleur compromis sensibilité/précision/consommation énergétique. Encore une fois, la consommation énergétique pour chaque irradiation étant contrôlée du fait que le nombre de paramètres d'irradiation est limité pour chaque cas d'irradiation et que ces paramètres d'irradiation tiennent compte des phénomènes d'interférences, même si la convergence n'est obtenue qu'après trois, quatre, cinq voire dix irradiations successives, la consommation énergétique globale peut être maîtrisée et notamment inférieure à celle qui serait nécessaire pour l'acquisition d'un spectre complet en fréquence d'irradiation et/ou en fréquence de modulation, et ce tout en contrôlant, voire en améliorant simultanément la précision de la mesure.

Selon un mode de réalisation du procédé de mesure, le procédé comprend au préalable :

I- au moyen d'un processeur et d'une base de données de configurations modèles comprenant des multiplets (configuration modèle du milieu stratifié cible, cas d'irradiation, paramètre d'intérêt) et un signal acoustique ou thermique détecté par la cellule de détection associé à chacun desdits multiplets, on génère un abaque de fréquences nodales pour :

- une pluralité de groupes de configurations modèles,
- et une pluralité de groupes de cas d'irradiation,

l'abaque de fréquences nodales comprenant une pluralité de multiplets nodaux, chaque multiplet nodal comprenant des caractéristiques communes à tous les éléments d'un groupe de configurations modèles du milieu stratifié cible et une pluralité de fréquences de modulation nodales associées,
et on stocke cet abaque de fréquences nodales dans la mémoire du capteur non invasif.

Grâce à cette disposition, on peut générer un abaque de fréquences nodales par simulation, donc in silico, dans un délai beaucoup plus court et couvrant une gamme de groupes de configurations modèles et de cas d'irradiations beaucoup plus complète que si l'abaque avait été généré manuellement. Cette disposition permet donc d'améliorer encore la précision du capteur. Elle permet aussi de réduire le coût et le temps de fabrication du capteur non invasif.

Selon un mode de réalisation, le procédé de mesure comprend :
II- un processeur apprend au moins un algorithme de modélisation inverse à partir de la base de données de configurations modèles et on stocke l'au moins un algorithme de modélisation inverse dans la mémoire du capteur non invasif.

Grâce à cette disposition, un ou plusieurs algorithmes de modélisation inverse peuvent être entraînés de manière automatisée, par exemple chacun adapté à une configuration modèle et un cas d'irradiation donné ou à un groupe de configurations modèles et de cas d'irradiation donnés, ce qui permet de gagner en précision à l'étape de résolution du problème inverse nécessaire pour déterminer une valeur du paramètre d'intérêt à partir du signal photoacoustique ou photothermique détecté.

Selon un mode de réalisation du procédé de mesure, au moins une partie des signaux acoustiques ou thermiques détectés par la cellule de détection associés aux multiplets stockés dans la base de données de configurations modèles sont simulés, c'est-à-dire qu'ils sont générés au moyen d'un dispositif informatisé de simulation.

Grâce à cette disposition, on peut générer un grand nombre de groupes de cas d'irradiation et de groupes de configuration par simulation, donc in silico, dans un délai beaucoup plus court et couvrant une gamme de groupes de configurations modèles et de cas d'irradiations beaucoup plus complète que ce qui est accessible par l'expérience in vivo. Cette disposition permet donc d'améliorer encore la précision du capteur. Elle permet aussi de réduire le coût et le temps de fabrication du capteur non invasif.

Selon un mode de réalisation du procédé de mesure la fréquence de modulation particulière déterminée à l'étape c) par le module d'adaptation est une moyenne pondérée d'au moins deux de ladite pluralité de fréquences de modulation nodales.

Ce mode de réalisation permet de tenir compte du fait que la fréquence de modulation correspondant au maximum de sensibilité par rapport au paramètre d'intérêt se trouve dans l'intervalle séparant deux fréquences de modulation nodales successives mais que la profondeur caractéristique de pénétration de l'onde lumineuse incidente décroît avec la fréquence de modulation. Il peut donc être intéressant, pour améliorer la précision du capteur non invasif, de ne pas choisir comme fréquence de modulation particulière la moyenne arithmétique de deux fréquences de modulation nodales mais plutôt une moyenne pondérée de ces fréquences de modulation nodales, notamment en attribuant un poids plus élevé à la fréquence de modulation nodale la plus basse.

Selon un mode de réalisation du procédé de mesure, le cas d'irradiation particulier pour la configuration modèle choisie déterminé par le module d'adaptation à l'étape c) comprend en outre au moins une fréquence de modulation nodale.

Cette disposition permet de recueillir, outre des informations sur le paramètre d'intérêt, des informations sur la configuration du milieu stratifié cible au moment de la mesure indépendamment du paramètre d'intérêt. En d'autres termes, les fréquences de modulation nodales permettent d'obtenir des informations sur les caractéristiques du milieu cible autres que le paramètre d'intérêt avec une précision améliorée.

L'invention porte aussi sur un capteur non invasif basé sur la détection photoacoustique ou photothermique configuré pour mesurer un paramètre d'intérêt dans un milieu stratifié cible comprenant :

- une source de lumière (1 1a),
- un dispositif de contrôle des paramètres d'irradiation de la source de lumière (1 1a),
- une cellule de détection (12) configurée pour détecter un signal acoustique ou thermique,
- une mémoire dans laquelle est stockée un abaque de fréquences nodales comprenant, pour :
- une pluralité de groupes de configurations modèles du milieu stratifié cible, comprenant chacun au moins deux configurations modèles du milieu stratifié cible ne différant les unes des autres que par le paramètre d'intérêt,
- et une pluralité de groupes de cas d'irradiation comprenant chacun au moins deux cas d'irradiation ne différant les uns des autres que par une fréquence de modulation d'une source de lumière, chaque cas d'irradiation comprenant un jeu de valeurs de paramètres d'irradiation, une pluralité de multiplets dits « nodaux », chaque multiplet comprenant des caractéristiques communes à tous les éléments d'un groupe donné de configurations modèles du milieu stratifié cible et une pluralité de fréquences de modulation nodales associées, pour lesquelles le signal acoustique ou thermique détecté par la cellule de détection en réponse à une irradiation par la source de lumière présente une corrélation inférieure à un seuil prédéterminé avec le paramètre d'intérêt,

le capteur non invasif comprenant en outre un module d'adaptation comprenant un processeur et adapté pour échanger des informations avec la cellule de détection et le dispositif de contrôle des paramètres d'irradiation de la source de lumière,

le module d'adaptation étant en outre configuré pour :

- choisir une configuration modèle du milieu stratifié cible initiale,
- déterminer dans l'abaque de fréquences nodales une pluralité de fréquences de modulation nodales associées à un groupe de configurations modèles auquel appartient la configuration modèle du milieu stratifié cible choisie, déterminer au moins une fréquence de modulation particulière sur la base de ladite pluralité de fréquences de modulation nodales et déterminer un cas d'irradiation particulier pour la configuration modèle d'irradiation choisie comprenant l'au moins une fréquence de modulation particulière ;
- transmettre un cas d'irradiation particulier au dispositif de contrôle des paramètres d'irradiation de la source de lumière,
- recevoir un signal acoustique ou thermique détecté par la cellule de détection,
- déterminer la valeur du paramètre d'intérêt sur la base du signal acoustique ou thermique détecté.

Salon un mode de réalisation du capteur non invasif, le processeur du module d'adaptation est de plus adapté pour :

- implémenter un algorithme de modélisation inverse recevant en entrée un cas d'irradiation comprenant un jeu de paramètres d'irradiation et un signal acoustique ou thermique et fournissant en sortie une configuration modèle du milieu stratifié cible et une valeur du paramètre d'intérêt ;
- déterminer au moyen de l'algorithme de modélisation inverse une configuration modèle du milieu stratifié cible en cours et une valeur du paramètre d'intérêt estimée sur la base d'un signal photoacoustique ou photothermique détecté reçu et d'un cas d'irradiation choisi,
- évaluer une configuration modèle d'irradiation du milieu stratifié cible choisie par comparaison avec une configuration modèle du milieu stratifié cible en cours,
- déterminer une nouvelle configuration modèle du milieu stratifié cible choisie en vue d'une irradiation lorsqu'il reçoit une configuration modèle du milieu stratifié cible en cours,
- déterminer la valeur du paramètre d'intérêt mesurée sur la base de la dernière valeur du paramètre d'intérêt estimé.

Selon un mode de réalisation du capteur non invasif, au moins deux des éléments parmi la source de lumière, le dispositif de contrôle des paramètres d'irradiation de la source de lumière, la cellule de détection configurée pour détecter un signal acoustique ou thermique, la mémoire dans laquelle est stocké un abaque de fréquences nodales et le module d'adaptation sont indépendants mécaniquement l'un de l'autre.

Ce mode de réalisation permet de former un capteur distribué, de sorte que l'encombrement ou le poids est réduit au niveau du milieu stratifié cible. Le module de simulation peut notamment être déporté pour une gestion distante et éventuellement partagée entre plusieurs utilisateurs. L'invention porte enfin sur un programme d'ordinateur comprenant des instructions qui conduisent le capteur non invasif suivant l'une des modes de réalisation précédents à exécuter les étapes du procédé de mesure suivant l'un des modes de réalisation décrits précédemment.

## BRÈVE DESCRIPTION DES DESSINS

Des modes de réalisation de l'invention seront décrits ci-dessous par référence aux dessins, décrits brièvement ci-dessous :

La figure 1 représente les éléments principaux d'un capteur non invasif 1 selon l'invention, le capteur non invasif 1 étant dans ce cas positionné en contact avec le milieu stratifié cible 2.

La figure 2 représente de manière simplifiée les différentes ondes thermiques transmises et réfléchies générées dans un milieu stratifié bicouche en réponse à une irradiation par un laser.

Les figures 3a et 3b représentent l'amplitude de l'onde thermique générée en réponse à une irradiation en fonction de la fréquence de modulation pour des concentrations en glucose variées, pour deux milieux stratifiés cibles différents.

La figure 4 représente une modélisation type d'un milieu cible stratifié de type peau.

La figure 5 représente les étapes d'un procédé de mesure suivant l'invention dans un mode de réalisation particulier, dans le cas où l'on souhaite mesurer la glycémie interstitielle d'un patient.

La figure 6 représente les étapes mise en oeuvre par le module de simulation 15 pour simuler un signal photoacoustique qui serait détecté par une cellule photoacoustique donnée en réponse à l'irradiation d'un milieu stratifié cible modélisé au moyen des paramètres de la configuration modèle CMk suivant les paramètres d'irradiation du cas d'irradiation Ij.

La figure 7 représente le fonctionnement d'un modèle inverse.

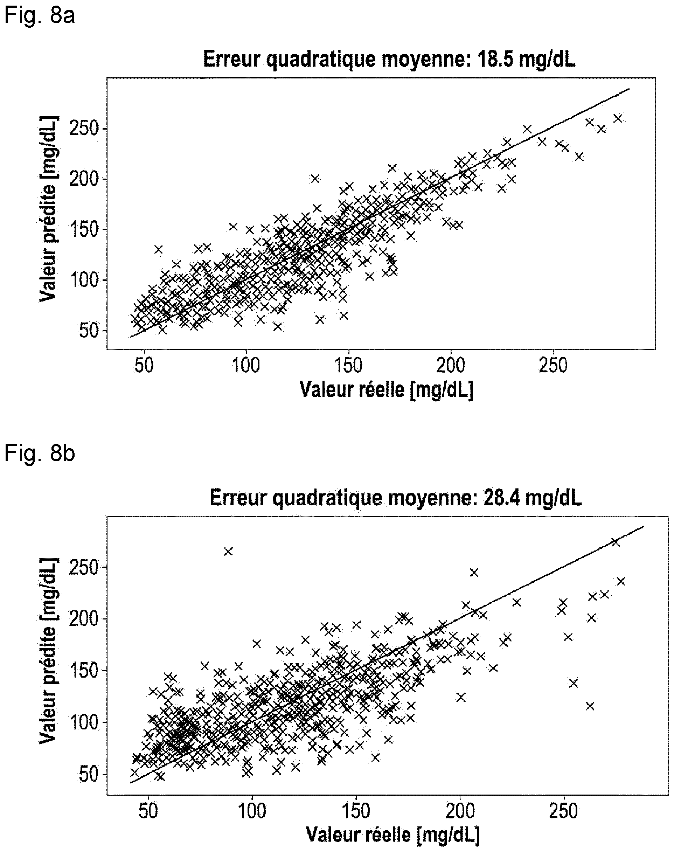

La figure 8a représente les valeurs prédites par le capteur non invasif en fonction des valeurs réelles, dans le cas où le milieu stratifié cible est la peau d'un patient, modélisée par un modèle bicouche (stratum corneum, épiderme). L'épaisseur de la couche supérieure (modélisant le stratum corneum) est égale à 14 $\mu$m et l'épaisseur de la couche inférieure (modélisant l'épiderme) est considérée comme infinie. La glycémie est nulle dans la couche supérieure. La couche supérieure comprend 20% d'eau et la couche inférieure comprend 60% d'eau. L'irradiation est réalisée avec les fréquences foptim = 1580 Hz, fnod1 = 950 Hz et fnod2 = 4750 Hz correspondant à cette configuration modèle.

La figure 8b représente les valeurs prédites par le capteur non invasif en fonction des valeurs réelles, dans le cas où le milieu stratifié cible est la peau d'un patient, modélisée par un modèle bicouche (stratum corneum, épiderme). L'épaisseur de la couche supérieure (modélisant le stratum corneum) est égale à 18 $\mu$m et l'épaisseur de la couche inférieure (modélisant l'épiderme) est considérée comme infinie. La glycémie est nulle dans la couche supérieure. La couche supérieure comprend 20% d'eau et la couche inférieure comprend 60% d'eau. L'irradiation est réalisée avec les fréquences foptim = 1580 Hz, fnod1 = 950 Hz et fnod2 = 4750 Hz ne correspondant pas à cette configuration modèle (épaisseur de la couche égale à 18 $\mu$m) mais correspondant à la configuration modèle utilisée pour la figure 8a.

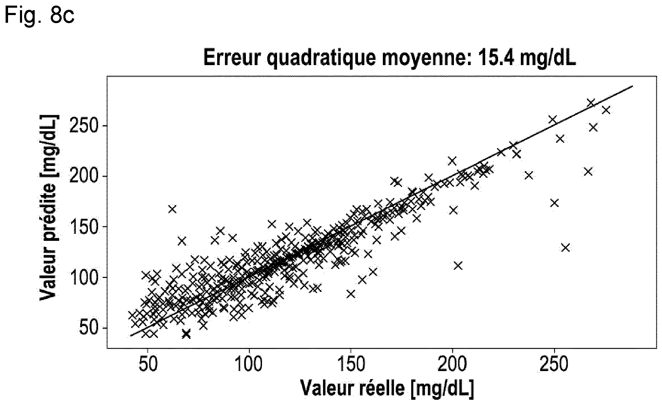

La figure 8c représente les valeurs prédites par le capteur non invasif en fonction des valeurs réelles, dans le cas où le milieu stratifié cible est la peau d'un patient, modélisée par un modèle bicouche (stratum corneum, épiderme). L'épaisseur de la couche supérieure (modélisant le stratum corneum) est égale à 18 $\mu$m et l'épaisseur de la couche inférieure (modélisant l'épiderme) est considérée comme infinie. La glycémie est nulle dans la couche supérieure. La couche supérieure comprend 20% d'eau et la couche inférieure comprend 60% d'eau. L'irradiation est réalisée avec les fréquences foptim = 1000 Hz, fnod1 = 600 Hz et fnod2 = 1300 Hz correspondant à cette configuration modèle.

## DESCRIPTION DÉTAILLÉE

L'invention concerne un capteur non invasif 1 d'un ou plusieurs paramètres d'un milieu stratifié cible 2, dont la structuration peut éventuellement évoluer au cours du temps. Le milieu stratifié cible 2 peut être, à titre d'exemple, un tissu d'un organisme humain ou animal, tel que la peau. Les paramètres à mesurer sont appelés dans la suite « paramètres d'intérêt ».

Un paramètre d'intérêt peut notamment être un paramètre physiologique dans le cas où le milieu stratifié est un tissu d'un être humain ou d'un animal.

Par exemple, le paramètre physiologique à mesurer est la glycémie, notamment la glycémie interstitielle. Il pourrait aussi s'agir de mesurer la teneur en eau dans une couche particulière de la peau ou encore la concentration en lactate d'une couche particulière. Ces exemples ne sont pas limitatifs.

Le capteur non invasif 1 peut être portable et il peut permettre le suivi en continu du ou des paramètres d'intérêt.

Le capteur non invasif 1 peut être basé sur la détection photoacoustique ou la détection photothermique.

Le procédé de mesure est particulièrement adapté pour améliorer la précision d'un capteur non invasif basé sur la détection photoacoustique indirecte, pour laquelle le capteur non invasif 1 détecte une onde acoustique générée dans le fluide environnant le milieu stratifié cible 2 en réponse à une irradiation, tout en contrôlant sa consommation énergétique ou encore pour réduire la consommation énergétique du capteur tout en contrôlant sa précision. Toutefois, il est tout-à-fait possible de mettre en oeuvre ce procédé sur un capteur non invasif basé sur la photothermie.

Pour simplifier la compréhension, l'exemple de la détection photoacoustique indirecte sera décrit plus en détails ci-après, mais la généralisation à un capteur basé sur la photothermie sera faite sans difficulté.

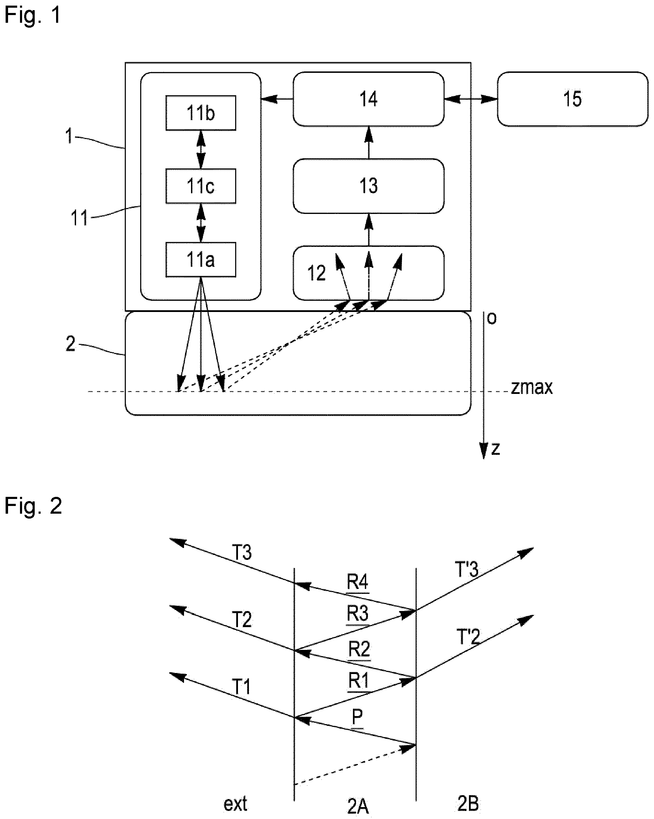

Le capteur non invasif 1 est représenté schématiquement sur la figure 1. Il comprend :

- un dispositif d'irradiation 11 comprenant une source de lumière 11a, un dispositif de modulation de l'intensité de cette source de lumière 11b, un dispositif de contrôle 11c d'au moins une fréquence de modulation à laquelle le dispositif de modulation d'intensité 11b module l'intensité d'une lumière émise par la source de lumière 11a ;
- au moins une cellule de détection photoacoustique 12 détectant un signal généré en réponse à une irradiation d'un milieu stratifié cible 2 par la lumière émise, par exemple détectant directement ou indirectement une onde thermique qui se propage dans un milieu stratifié cible 2 ;
- un module de traitement du signal 13 configuré pour recevoir et traiter des données de l'au moins une cellule de détection 12 ;
- un module d'adaptation 14 des paramètres d'irradiation et du modèle de calibration ;
- pour certains modes de réalisation, un module de simulation 15, distant ou embarqué avec les autres éléments du capteur non invasif 1.

Dans un mode de réalisation particulier, la source de lumière 11a émet un faisceau laser modulé en intensité à au moins une longueur d'onde particulière vers le milieu stratifié cible 2.

L'au moins une longueur d'onde peut être choisie en fonction d'un des paramètres d'intérêt. A titre d'exemple, le nombre d'onde 1034 cm$^{-1}$, correspondant à un pic d'absorption du glucose, pourra être pertinent si le capteur non invasif 1 est un capteur de glycémie.

La source de lumière 11a peut notamment être une diode électroluminescente (DEL) ou une puce laser. La source de lumière 11a peut, en outre ou en variante, comprendre un laser à cascade quantique (QCL) émettant dans la région de l'infrarouge moyen (MIR-QCL), un laser ICL (« Interband Cavity Laser »), un laser à cavité interne ou externe, un laser GaSb. Ces exemples ne sont pas limitatifs. La source de lumière 11a pourra être choisie en fonction du milieu stratifié cible 2 et/ou des paramètres d'intérêt.

Le capteur non invasif 1 peut comprendre plusieurs sources de lumière 11a différentes et/ou identiques.

Le capteur non invasif 1 comprend également les circuits associés à la (ou aux) sources de lumière 11a et au moins un dispositif de contrôle 11c configuré pour contrôler la fréquence à laquelle au moins un dispositif de modulation d'intensité 11b module l'intensité d'au moins une source de lumière 11a, de sorte que l'intensité de la lumière émise par la source de lumière 11a soit modulée à une fréquence de modulation réglable.

La fréquence de modulation de l'intensité d'une source de lumière 11a donnée est appelée fmod dans la suite de la description.

La source de lumière 11a peut être modulée en intensité par tout moyen électrique ou mécanique connu.

La lumière irradiant le milieu stratifié cible 2 peut être émise de manière continue ou pulsée par une source de lumière 11a donnée.

La lumière incidente sur le milieu stratifié cible 2, émise par la (ou les) source(s) de lumière 11a modulée(s) en intensité, se propage jusqu'au milieu stratifié cible 2 puis à travers ce milieu stratifié cible 2 (phénomène symbolisé par des flèches en traits pleins sur la figure 1). Elle est alors progressivement absorbée par les différents constituants de ce milieu stratifié cible 2, sur une profondeur caractéristique, notée zmax, qui dépend de la structure du milieu stratifié cible 2 et de sa composition physico-chimique.

L'absorption de l'énergie lumineuse provoque un échauffement local du milieu stratifié cible 2. En conséquence, une onde thermique de fréquence égale à la fréquence de modulation de la source de lumière se propage dans le milieu stratifié cible 2 (phénomène symbolisé par des flèches en traits pointillés sur la figure 1), notamment vers la surface du milieu stratifié cible 2. Cette onde thermique donne naissance dans le milieu gazeux extérieur à la cible à une onde de pression de même fréquence, qui se propage dans ce milieu fluide, par exemple gazeux, environnant le milieu stratifié cible 2 et notamment dans la cellule de détection photoacoustique 12 (phénomène symbolisé par des flèches en pointillés alternés sur la figure 1). A ce stade, le phénomène d'interférences qui nous intéresse n'est pas décrit. Il le sera plus loin.

La cellule de détection 12 comprend, dans le cas de la détection photoacoustique, une chambre remplie d'un gaz (par exemple de l'air) à travers lequel se propage l'onde acoustique. La cellule de détection 12 comprend aussi un ou plusieurs capteurs appropriés placés dans cette chambre, par exemple face au milieu stratifié cible 2. Il s'agit par exemple d'un ou plusieurs capteurs électroacoustiques configurés pour convertir la pression de l'onde acoustique en un signal électrique, tel qu'un microphone ou encore un transducteur piézoélectrique.

Chaque capteur électroacoustique est fonctionnellement connecté au module de traitement de signal 13.

Le module de traitement de signal 13 peut comprendre un convertisseur analogique-numérique configuré pour convertir le signal électrique analogique du capteur électroacoustique en un signal numérique.

Le module de traitement du signal 13 peut comprendre un dispositif de détection synchrone adapté pour démoduler et extraire le signal d'intérêt du signal détecté.

Le module de traitement de signal 13 comprend éventuellement un amplificateur opérationnel connecté de manière opérationnelle au convertisseur analogique-numérique et configuré pour amplifier le signal électronique dérivé de la réponse acoustique du milieu stratifié cible 2 transmis par un capteur électroacoustique.

Dans des exemples de modes de réalisation, le convertisseur analogique-numérique est fonctionnellement connecté à un processeur de signal numérique pour le traitement du signal numérique.

Le capteur non invasif 1 selon l'invention comprend en outre un module d'adaptation 14 des paramètres d'irradiation et éventuellement du modèle de calibration, ainsi qu'un module de simulation 15. Ces deux éléments seront décrits plus loin, après que l'on ait précisé l'origine du problème technique à résoudre, à savoir fournir un capteur non invasif 1 basé sur la détection photoacoustique ou photothermique de précision et/ou de consommation contrôlées grâce à la prise en compte des phénomènes d'interférences.

## Phénomène d'interférences d'ondes thermiques

Le milieu stratifié cible 2 comprenant au moins deux couches superposées présentant des caractéristiques physico-chimiques différentes, il comporte au moins une interface au niveau de laquelle l'onde incidente peut se réfléchir.

Dans un cas simple, représenté sur la figure 2, dans lequel le milieu stratifié cible 2 comprend deux couches 2A et 2B, la couche supérieure 2A est en contact avec le milieu extérieur au niveau de l'interface 2A/ext et avec la couche inférieure au niveau de l'interface 2A/2B.

La couche supérieure 2A est par exemple le stratum corneum de la peau d'un patient et la couche inférieure 2B est par exemple l'épiderme de la peau d'un patient dans le cas d'un capteur non invasif 1 adapté pour une mesure de glycémie.

Même dans ce cas simple comme celui-ci, la description extensive de l'onde thermique générée dans le milieu stratifié cible 2 nécessite de faire appel à des modèles complexes, tels que le modèle dit « de Hu », décrit dans Hu, Hanping et al. "Generalized theory of the photoacoustic effect in a multilayer material." Journal of Applied Physics 86 (1999): 3953-3958. Ce modèle comprend de manière implicite une partie des effets d'interférences thermiques, mais ne tient pas compte des interférences dues aux réflexions multiples sur les interfaces. D'autres modèles proposent de tenir compte de ces réflexions multiples, notamment celui décrit par Cao, J. (2000) ; Interferential formulization and interprétation of the photoacoustic effect in multilayered cells ; Journal of Physics D: Applied Physics, 33(3), 200.

Quel que soit le modèle choisi, le phénomène d'interférences a un impact significatif sur l'amplitude du signal détecté et en conséquence sur la précision de la mesure. Cet impact dépend de la structuration du milieu stratifié cible 2.

Pour comprendre de manière grossière cet impact, on peut se baser sur la figure 2, qui n'a de valeur que pédagogique. Dans ce cas, une onde thermique dite « primaire » P est générée par l'irradiation (symbolisée par la flèche pointillée) au niveau de l'interface 2A/2B. Cette onde primaire P se propage notamment en direction de l'interface 2A/ext, sur laquelle elle se réfléchit partiellement, ce qui donne lieu à une première onde transmise dans le milieu extérieur T1 et une onde réfléchie R1. L'onde réfléchie R1 peut-elle-même se réfléchir sur l'interface 2A/2B pour donner lieu à une onde transmise T'2 et une onde réfléchie R2. L'onde réfléchie R2 va elle-même donner lieu, après propagation vers l'interface 2A/ext à une deuxième onde transmise dans le milieu extérieur T2 et une onde réfléchie R3, et ainsi de suite.

Toutes les ondes transmises dans le milieu extérieur T1, T2,..., sont susceptibles d'interférer entre elles de sorte que l'amplitude de l'onde acoustique qui se forme dans le milieu extérieur en réponse à l'irradiation en est affectée. Dans ce cas, on peut montrer que l'amplitude de l'onde thermique au niveau de l'interface 2A/air a pour expression :

$$\theta = T_{2A/ext} R_{2A/2B} \frac{1}{1 - R_{2A/2B} R_{2A/ext} \exp\left(-2(1+j)l_2 \sqrt{\frac{\omega}{2\alpha_2}}\right)}$$

Formule 1

où :

- $R_{2A/2B} = \frac{\varepsilon_{2A} - \varepsilon_{2B}}{\varepsilon_{2A} + \varepsilon_{2B}}$ est le coefficient de réflexion thermique à l'interface 2A/2B

- $R_{2A/ext} = \frac{\varepsilon_{2A} - \varepsilon_{ext}}{\varepsilon_{2A} + \varepsilon_{ext}}$ = est le coefficient de réflexion thermique à l'interface 2A/ext

- $T_{2A/ext} = \frac{2\varepsilon_{ext}}{\varepsilon_{2A} + \varepsilon_{ext}}$ = est le coefficient de transmission thermique à l'interface 2A/ext

- $\alpha_2$ est la diffusivité thermique du milieu 2A
- $l_2$ est l'épaisseur de la couche 2A
- $\varepsilon_{2A}$, $\varepsilon_{2B}$, $\varepsilon_{ext}$ sont les effusivités thermiques respectives de la couche 2A, la couche 2B et du milieu extérieur.

Les effusivités thermiques de même que l'épaisseur de la couche 2A peuvent, dans le cas de notre milieu stratifié cible 2, varier au cours du temps. Notamment, dans le cas où le milieu stratifié cible 2 est la peau et où le capteur non invasif 1 est un capteur de glycémie, l'épaisseur des couches de peau, leurs concentrations en eau ainsi que leurs concentrations en glucose, qui sont susceptibles d'affecter les différentes effusivités thermiques, évoluent ainsi au cours du temps, de sorte qu'en un même site de détection, le signal photoacoustique ou photothermique en est affecté.

On comprend donc que la précision et la sensibilité de la mesure du paramètre d'intérêt, par exemple de la glycémie, sur la base du signal photoacoustique et photothermique dépend de l'information dont on dispose concernant ces phénomènes d'interférences.

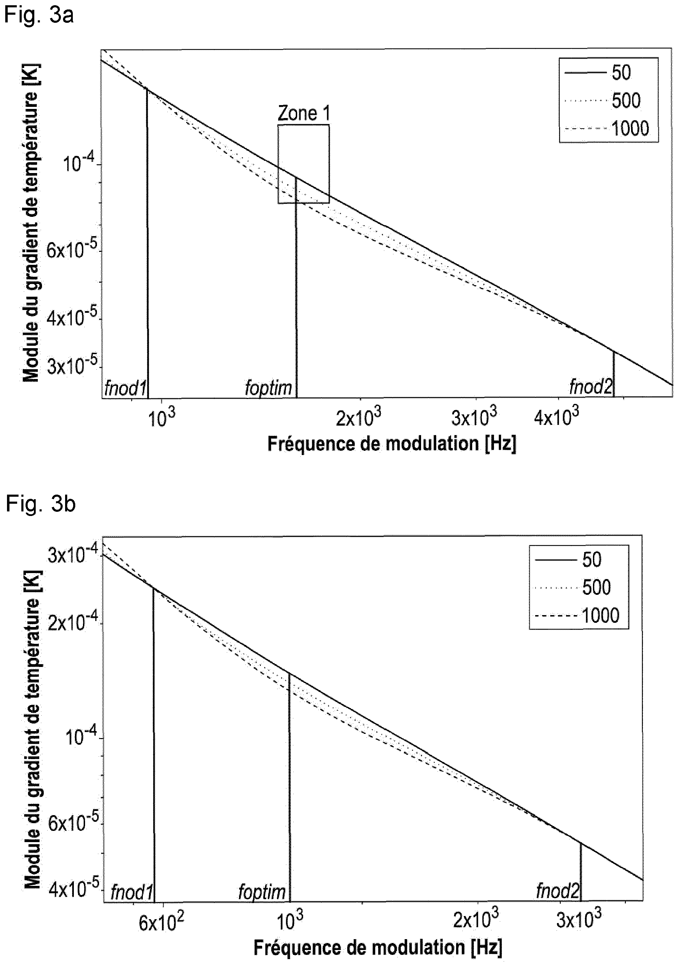

Nous insistons ici sur la difficulté d'accéder à cette information dans le cas d'un milieu stratifié cible 2 évolutif, dont les caractéristiques des différentes strates changent au cours du temps. Pour résoudre ce problème, les inventeurs ont notamment développé un module de simulation 15 qui sera décrit en détails plus loin et qui permet entre autres de simuler le signal photothermique associé à une irradiation dont toutes les caractéristiques (puissance, longueur(s) d'onde(s), fréquence(s) de modulation...) sont connues, le milieu stratifié cible 2 étant lui aussi modélisé par un milieu multicouches dont les caractéristiques (épaisseurs, compositions...) sont connues, sur la base d'un modèle analytique multiphysique tenant compte au moins implicitement des phénomènes d'interférences thermiques. Ce module de simulation 15 a permis de réaliser des expériences in silico extensives, dont une partie des résultats sont présentés sur les figures 3a et 3b.

Dans le cas de la figure 3a, le milieu stratifié cible 2 est la peau, modélisée par un modèle bicouche (stratum corneum, épiderme). L'épaisseur de la couche supérieure (modélisant le stratum corneum) est égale à 14 μm, celle de la couche inférieure (modélisant l'épiderme) est considérée comme infinie. La glycémie est nulle dans la couche supérieure. La couche supérieure comprend 20% d'eau et la couche inférieure comprend 60% d'eau.

Le modèle de peau est irradié (de manière simulée) avec un laser de nombre d'onde 1034 cm⁻¹ modulé en intensité à une fréquence de modulation qui est portée sur l'axe des abscisses. L'amplitude de l'onde thermique réponse simulée générée en réponse à une irradiation est représentée en ordonnée en fonction de la fréquence de modulation pour différentes valeurs de glycémie de la couche inférieure (50 mg/L ; 500 mg/L et 1000 mg/dL).

De manière inattendue, les inventeurs ont observé l'existence de fréquences de modulation dites « nodales » à proximité desquelles l'amplitude de l'onde thermique est indépendante du paramètre d'intérêt, en l'occurrence la glycémie.

Dans le cas de la figure 3a, on observe ainsi deux fréquences de modulation nodales, à savoir fnod1 = 950 Hz et fnod2 = 4 750 Hz, mais il en existe encore d'autres à l'extérieur de la gamme de fréquences de modulation représentée sur la figure 3a. Ces fréquences de modulation nodales peuvent s'expliquer par le phénomène d'interférences d'ondes thermiques au sein du milieu stratifié cible 2.

Les fréquences de modulation nodales correspondent à une onde réponse générée dans le milieu dont l'amplitude est faiblement corrélée au paramètre d'intérêt, voire indépendante du paramètre d'intérêt (dans ce cas : la glycémie interstitielle), donc le signal détecté à une telle fréquence de modulation ne peut pas servir pour la détermination de ce paramètre d'intérêt. Dans le cas de la figure 3b, le milieu stratifié cible 2 est la peau, modélisée par un modèle bicouche (stratum corneum, épiderme). L'épaisseur de la couche supérieure (modélisant le stratum corneum) est cette fois-ci égale à 18 μm, celle de la couche inférieure (modélisant l'épiderme) est considérée comme infinie. La glycémie est nulle dans la couche supérieure. La couche supérieure comprend 20% d'eau et la couche inférieure comprend 60% d'eau.

Le modèle de peau est irradié (de manière simulée) avec un laser de nombre d'onde 1034 cm⁻¹ modulé en intensité à une fréquence de modulation qui est portée sur l'axe des abscisses. L'amplitude de l'onde thermique réponse simulée générée en réponse à une irradiation est représentée en ordonnée en fonction de la fréquence de modulation pour différentes valeurs de glycémie de la couche inférieure (50 mg/L ; 500 mg/L et 1000 mg/L).

On observe à nouveau des fréquences de modulation nodales, en l'occurrence fnod1 = 600 Hz et fnod2 = 3000 Hz, mais ces fréquences de modulation nodales sont différentes de celles obtenues dans la situation de la figure 3a, comme attendu du fait de la modification de l'épaisseur de la couche 2A de 14 μm à 18 μm.

De manière générale, les expériences extensives in silico réalisées par les inventeurs leur ont donc permis d'observer les faits suivants :

- il existe des fréquences de modulation nodales, à proximité desquelles l'amplitude de l'onde thermique générée en réponse à l'irradiation est faiblement corrélée au paramètre d'intérêt, voire indépendante du paramètre d'intérêt. On remarquera que si on agrandit l'échelle des figures 3a et 3b, il ne s'agit pas réellement d'une fréquence de modulation nodale unique mais d'une gamme de fréquences restreinte dans laquelle l'amplitude thermique varie très peu quand le paramètre d'intérêt varie ;
- les valeurs des fréquences de modulation nodales dépendent des caractéristiques du milieu stratifié cible 2, à savoir notamment des épaisseurs et des compositions des différentes couches qui le constituent.

Il est très difficile de réaliser suffisamment d'observations in vivo pour obtenir de telles courbes. Ces observations n'ont donc été rendues possibles que grâce au simulateur développé par les inventeurs. Ces observations sont donc nouvelles et permettent d'envisager un capteur non invasif 1 tenant compte des phénomènes d'interférences.

Les inventeurs ont donc conçu, sur la base de ces observations, un capteur non invasif 1 et un procédé de détection d'un paramètre d'intérêt amélioré par l'art antérieur, que nous allons maintenant décrire plus en détails.

Dans le capteur non invasif 1, les fréquences de modulation nodales peuvent être mises à profit dans le contexte du capteur non invasif 1 grâce au module d'adaptation 14 qui le caractérise et, dans un mode de réalisation particulier au module de simulation 15.

## Module d'adaptation 14

Le module d'adaptation 14 est un dispositif informatisé comprenant au moins un processeur :

- qui peut échanger des informations le cas échéant avec le module de simulation 15 décrit plus loin ;
- qui peut recevoir des informations de la cellule de détection 12 et/ou du module de traitement du signal 13 le cas échéant ;
- et qui peut transmettre des informations au(x) dispositif(s) de contrôle 11c d'au moins un paramètre d'irradiation de la source de lumière 11a (par exemple une fréquence de modulation à laquelle le dispositif de modulation d'intensité 11b module l'intensité d'une lumière émise par la source de lumière 11a, le nombre d'onde d'une lumière émise par la source de lumière 11a, la puissance lumineuse de la source de lumière 11a, ...).

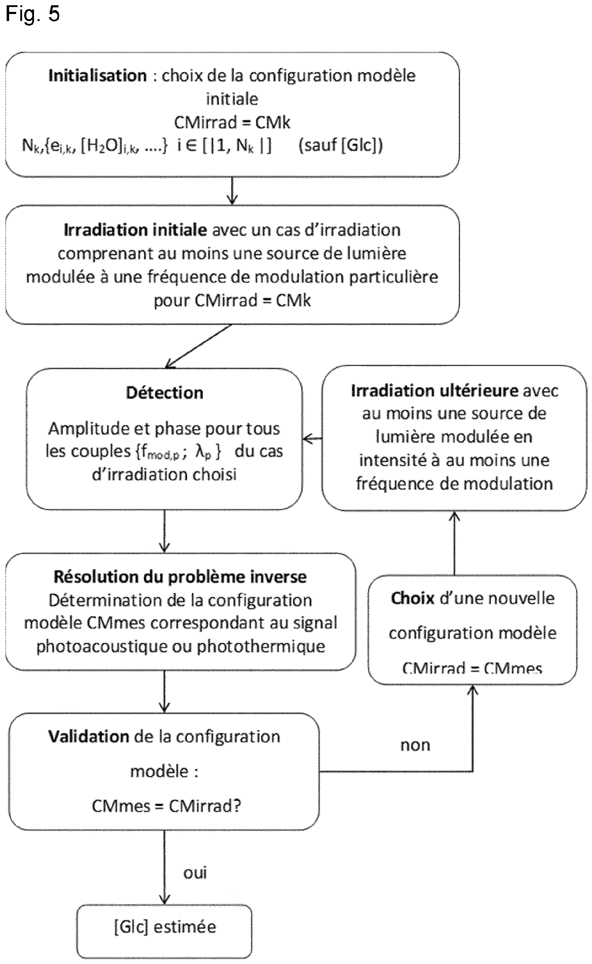

Les étapes mises en oeuvre par un capteur non invasif 1 comprenant un module d'adaptation 14 pour une mesure du paramètre d'intérêt sont représentées schématiquement sur la figure 5 dans un mode de réalisation particulier et dans le cas où le milieu stratifié cible 2 est la peau et le paramètre d'intérêt est la glycémie interstitielle.

Le capteur non invasif 1 comprend en outre une mémoire de stockage d'une base de données de configurations modèles, d'un abaque de fréquences nodales et d'un ou plusieurs modèles inverses qui sont décrits ci-après.

Cette mémoire de stockage peut être distribuée et/ou partagée dans/avec le module d'adaptation (14) et/ou le module de simulation (15).

Le module d'adaptation 14 réalise les étapes du procédé qui permettent de choisir les paramètres d'irradiation pour la mesure sur la base de l'abaque de fréquences nodales et de déterminer le modèle inverse le plus adapté, c'est-à-dire le plus précis, pour le calcul du paramètre mesuré à partir du signal détecté pour cette mesure particulière. Nous décrirons ces étapes après avoir décrit les étapes permettant de générer la base de données de configurations modèles et l'abaque de fréquences nodales.

La base de données de configurations modèles et l'abaque de fréquences nodales peuvent être générés à partir d'un module de simulation 15, embarqué dans le capteur 1 ou distant. Dans le cas où le module de simulation 15 est distant, le capteur non invasif 1 comprend des moyens de communication pour que le module de simulation 15 et le module d'adaptation 14 échangent des données.

## Module de simulation 15

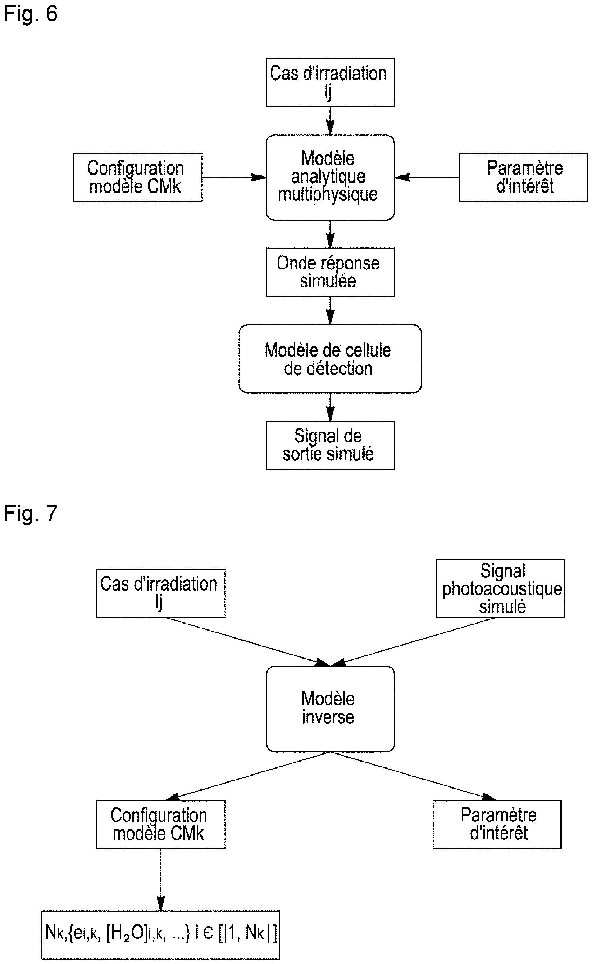

Le module de simulation 15 est un dispositif informatisé configuré pour générer un ensemble de configurations modèles correspondant (ou encore modélisant ou décrivant) chacune à un état particulier du milieu stratifié cible 2, un ensemble de cas d'irradiation (comprenant chacun au moins un paramètre d'irradiation tel qu'une puissance lumineuse, une longueur d'onde et une fréquence de modulation) du milieu stratifié cible 2 et les signaux photoacoustiques (ou le cas échéant photothermiques) théoriquement détectés en réponse à chaque cas d'irradiation pour chaque configuration modèle CMk du milieu stratifié cible 2 à partir de modèles analytiques du milieu stratifié cible 2 et de la cellule de détection photoacoustique (ou le cas échéant photothermique) 12, comme représenté sur la figure 6.

Ce module de simulation 15 est particulièrement pertinent dans notre cas, pour lequel le milieu stratifié cible 2 est évolutif au cours du temps et/ou stratifié. Dans ce cas, le milieu stratifié cible 2 adopte en effet au cours du temps des configurations réelles différentes (une ou plusieurs concentrations varient au sein d'une ou plusieurs couches du milieu stratifié cible 2, une ou plusieurs dimensions telles que, par exemple, l'épaisseur d'une des couches du milieu stratifié cible 2 varient) qui peuvent chacune être modélisée par une configuration modèle particulière.

a) Modèle analytique multiphysique du milieu stratifié cible 2

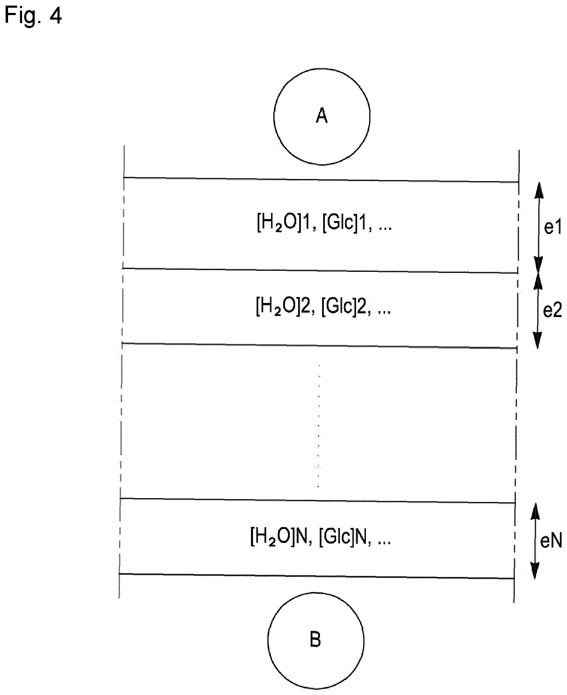

Le milieu stratifié cible 2 à analyser est modélisé comme représenté sur la figure 4. Le milieu cible 2 sépare un milieu extérieur A d'un milieu intérieur B et est composé d'une succession de N couches dont les interfaces sont par exemple supposées localement planes.

Chaque couche i ( $i \in [\![1, N]\!]$ ) est décrite par le(s) paramètres d'intérêt et un certain nombre de paramètres explicites (appelés paramètres de niveau 1 car leurs valeurs seront fournies en entrée du module de simulation 15 pour chaque simulation) appropriés pour le milieu stratifié cible 2 considéré. A titre d'exemple, si le milieu stratifié cible 2 est la peau et que l'on cherche à mesurer une concentration en glucose dans une couche j de ce milieu cible 2, le milieu cible 2 sera décrit par son nombre de couches N, la concentration [Glc]j d'intérêt, et chaque couche i peut être décrite pour la modélisation par son épaisseur ei, sa concentration en eau [H2O]i. On peut éventuellement tenir compte des concentrations en glucose [Glc]i pour i ≠ j. Dans ce cas les valeurs de $e_i$ ( $i \in [\![1, N]\!]$ ), les concentrations [H2O]$_i$ ( $i \in [\![1, N]\!]$ ) et les concentrations [Glc]i pour ( $i \in [\![1, N]\!]$ , i ≠ j) sont les paramètres de niveau 1.

La liste des paramètres de niveau 1 peut être enrichie si l'on souhaite réaliser une modélisation plus précise. Notamment, les concentrations d'autres composants de la peau tels que les graisses, le lactate, l'oxygène, etc., peuvent être incluses dans la liste des paramètres de niveau 1 décrivant une couche de la peau.

Toujours pour l'exemple de la peau, on peut envisager de tenir compte de la couleur de la peau, de l'âge du patient, ou de tout autre paramètre anthropométrique, de manière à élargir ou restreindre l'espace des modèles possibles.

Des paramètres non explicites du modèle de milieu stratifié cible 2 (appelés paramètres de niveau 2 car non fournis en entrée du module de simulation 15) peuvent être calculés au moyen de modèles analytiques. Par exemple, la conductivité thermique, la capacité thermique, la densité ou encore le coefficient d'absorption à chaque longueur d'onde de chaque couche du milieu stratifié cible 2 peuvent être déduits des paramètres de niveau 1 et d'équations connues. Le paramètre d'intérêt jouant un rôle particulier, il n'est pas inclus dans la liste des paramètres de niveau 1. Suivant l'étape du procédé, ce paramètre d'intérêt sera connu ou non : sa valeur est connue pour procéder aux simulations au moyen du module de simulation 15, mais elle est bien sûr inconnue dans le cas d'une mesure réelle avec le capteur non invasif 1.

Le nombre de couches N du milieu stratifié cible 2 peut aussi être une variable du modèle. Toujours dans l'exemple de la peau, suivant les situations physiologiques, N peut ainsi être supérieur ou égal à deux. Ainsi, pour certaines situations physiologiques, la peau pourra être correctement décrite par deux couches, la première correspondant au stratum corneum, dont la concentration en glucose peut par exemple être faible, et la deuxième au reste de la peau, la concentration en glucose de la seconde couche pouvant être assimilée à la concentration en glucose interstitielle à mesurer.

Pour d'autres situations, un modèle à trois couches ou plus sera plus adapté. Dans ce dernier cas, la concentration en eau d'une couche pourra par exemple augmenter avec la profondeur à laquelle se trouve cette couche.

N peut donc ne pas être une constante.

Dans le cas de la peau, le milieu extérieur A est en général l'atmosphère entourant le patient, qui remplit aussi la cellule de détection photoacoustique.

Le module de simulation 15 implémente un modèle de milieu stratifié 2 analytique multiphysique basé par exemple sur des équations physiques et/ou chimiques telles que, à titre d'exemple non limitatif, les équations de Beer-Lambert pour l'absorption optique et les équations thermodynamiques de la chaleur (loi de Fourier et lois de conservation).

Une configuration modèle CMk (k entier positif) de milieu stratifié cible 2 correspond à (ou encore modélise) un état particulier du milieu stratifié cible 2 donné. Cet état particulier est supposé correctement représenté par la donnée du nombre de couches N et des valeurs des paramètres de niveau 1 pour chaque couche.

Pour chaque configuration modèle CMk, le modèle analytique multiphysique permet, si le paramètre d'intérêt est de plus connu, de simuler l'onde thermique générée à l'interface couche 1/ milieu extérieur A (interface 1/A) en réponse une irradiation par une source de lumière 11a dont les paramètres d'irradiation sont connus, à savoir par exemple la fréquence de modulation fmod, la longueur d'onde λ et la densité surfacique de puissance.

En variante, le modèle analytique multiphysique permet de simuler l'onde de pression générée dans le milieu extérieur A.

Dans les deux cas, le signal obtenu en sortie d'un processeur implémentant le modèle analytique multiphysique est appelé « onde réponse simulée ».

L'onde réponse simulée peut être fournie en entrée d'un processeur implémentant le modèle de cellule de détection.

b) <u>Modèle de cellule de détection</u>

Le capteur non invasif 1 basé sur la détection photoacoustique ou photothermique comprend une cellule de détection photoacoustique (respectivement photothermique) 12 configurée pour détecter et analyser l'onde de pression (respectivement l'onde thermique) générée dans le milieu extérieur A lorsque l'onde thermique générée dans le milieu stratifié cible 2 en réponse à l'irradiation parvient à l'interface 1/A.

L'ensemble de la cellule de détection 12 peut être modélisé analytiquement. A partir d'une onde réponse simulée par le modèle analytique multiphysique, qui serait théoriquement reçue en entrée de la cellule de détection 12, le modèle de cellule de détection 12 permet de prédire le signal de sortie de la cellule de détection 12.

Différents modèles peuvent être envisagés.

Ainsi, dans le cas de la détection photoacoustique indirecte, les paramètres du modèle de la cellule de détection 12, appelés paramètres de cellule dans la suite, peuvent comprendre ses dimensions (par exemple la taille de l'évent, la hauteur de la cellule,...), des paramètres d'état thermodynamiques (température, pression atmosphérique, humidité relative ou absolue, ...). La cellule de détection photoacoustique peut notamment être modélisée au moyen d'un circuit RLC équivalent. A titre d'exemple, un modèle dérivé du modèle décrit dans Dehe, Alfons et al. "The Infineon Silicon MEMS Microphone." (2013) peut convenir.

Il est possible d'inclure dans ce modèle de cellule de détection photoacoustique un modèle de l'étape de traitement du signal effectuée par le module de traitement du signal 13 le cas échéant, de manière à générer, à partir de chaque onde réponse simulée générée par le processeur qui implémente le modèle multiphysique analytique, le signal théoriquement obtenu en sortie de cellule de détection photoacoustique (et le cas échéant après traitement du signal par le module de traitement du signal 13) qui lui correspond.

Le processeur du module de simulation 15 peut être configuré pour implémenter le modèle de cellule de détection photoacoustique.

Grâce au modèle analytique multiphysique de milieu stratifié cible 2 et au modèle de cellule de détection 12, on dispose donc d'un modèle analytique global qui permet à partir de la donnée de la configuration modèle CMk de milieu stratifié cible 2 et du cas d'irradiation Ij, à condition de fournir en outre le paramètre d'intérêt (qui peut effectivement être choisi puisqu'il s'agit d'une simulation), de prévoir le signal attendu en sortie de la cellule de détection 12 ou, le cas échéant, du module de traitement du signal 13. Ceci est représenté sur la figure 6.

Le modèle analytique multiphysique est choisi pour tenir compte, au moins de manière implicite, des interférences d'ondes thermiques. Il peut, dans un mode de réalisation particulier, tenir compte de ces interférences de manière explicite.

Il peut encore, en variante, tenir compte de tout ou partie des réflexions multiples aux interfaces du milieu stratifié cible 2.

En résumé, le module de simulation 15 reçoit donc en entrée les paramètres de la configuration modèle CMk de milieu stratifié cible 2, c'est-à-dire le nombre de couches N du milieu cible 2 et les paramètres de niveau 1 pour chaque couche, ainsi que le paramètre d'intérêt et les paramètres d'irradiation du cas d'irradiation Ij. En sortie, le module de simulation 15 fournit le signal théoriquement attendu en sortie de la cellule de détection 12 ou le cas échéant, le signal simulé traité théoriquement attendu en sortie du module de traitement du signal 13 pour la configuration modèle CMk de milieu cible 2 choisie, appelé signal de sortie simulé.

Le signal de sortie simulé peut être stocké en mémoire sous la forme d'un spectre de Fourier.

Les multiplets {configuration modèle CMk de milieu cible 2, cas d'irradiation Ij, paramètre d'intérêt, amplitudes et phases des composantes du signal de sortie simulé} peuvent être stockés dans une base de données de configurations modèles.

**Base de données de configurations modèles**

On peut donc générer, éventuellement de manière automatisée et/ou aléatoire, un grand nombre de configurations modèles CMk, chaque configuration modèle CMk correspondant à un nombre de couches N et un jeu de paramètres de niveau 1, et optionnellement à une valeur ou une gamme de valeurs du paramètre d'intérêt, décrivant une situation particulière du milieu stratifié cible 2 d'intérêt.

Pour chaque configuration modèle CMk, on peut générer, éventuellement de manière automatisée et/ou aléatoire un grand nombre de cas d'irradiation Ij, chaque cas d'irradiation correspondant à un jeu de paramètres d'irradiation décrivant les paramètres de la (des) sources de lumière 11a utilisée(s) pour l'irradiation.

Un cas d'irradiation Ij peut donc comprendre une ou plusieurs fréquences de modulation de l'intensité d'un ou plusieurs lasers, la longueur d'onde de chacun de ces lasers et optionnellement la puissance irradiée par chaque laser.

Au moyen du module de simulation 15, on calcule l'amplitude et la phase de chaque composante du signal de sortie simulé obtenu en sortie du processeur du module de simulation 15 implémentant le modèle analytique global comprenant en cascade le modèle analytique multiphysique et le modèle de cellule de détection pour chaque configuration modèle CMk pour chaque cas d'irradiation Ij, une valeur du paramètre d'intérêt étant de plus fournie.

Les cas d'irradiation Ij peuvent être les mêmes pour plusieurs configurations modèles CMk différentes, et éventuellement plusieurs valeurs du paramètre d'intérêt, ou différents d'une configuration modèle CMk à une autre et/ou d'une valeur du paramètre d'intérêt à une autre. Une fois les simulations réalisées, on peut stocker dans une base de données de configurations modèles l'ensemble de ces configurations modèles CMk, cas d'irradiations, paramètres d'intérêt et signaux photoacoustiques (ou le cas échéant photothermiques) simulés associés sous forme de multiplets {configuration modèle CMk, cas d'irradiation Ij, paramètre d'intérêt, amplitude et phase des composantes du signal de sortie simulé}

La génération des configurations modèles CMk et/ou des cas d'irradiation Ij peut ne pas être complètement aléatoire.

La génération des configurations modèles CMk peut, entre autres, être fondée sur des considérations physiologiques pour restreindre l'espace des possibles à des configurations modèles physiologiquement réalistes. On peut par exemple limiter les épaisseurs possibles de la première couche de la peau à la gamme [8 $\mu$m, 40 $\mu$m] qui est effectivement observée expérimentalement, et limiter les concentrations en eau de cette couche à une gamme restreinte pour chaque épaisseur, la concentration en eau du stratum corneum étant corrélée avec son épaisseur.

La génération des cas d'irradiation peut notamment tenir compte des limitations des sources de lumière 11a disponibles pour un capteur non invasif 1 donné en longueur d'onde et/ou en puissance, ou encore des gammes de fréquences de modulation pertinentes pour le type de milieu stratifié cible 2 à analyser, ou des longueurs d'onde pertinentes pour le ou les paramètres d'intérêt.

En variante, la base de données de configurations modèles peut ne comprendre que des multiplets {configuration modèle CMk, cas d'irradiation Ij, paramètre d'intérêt, amplitudes et phases des composantes du signal mesuré réellement} obtenus par des expériences en situation réelles ou comprendre à la fois de tels multiplets obtenus en situation réelle et des multiplets obtenus par simulation.

## Modèle inverse

A partir de la base de données de configurations modèles, on peut entraîner dans le module de simulation 15 un ou plusieurs modèles d'intelligence artificielle.

Le modèle d'intelligence artificielle, après apprentissage, est capable de résoudre le problème inverse, c'est-à-dire de retrouver le (ou les) paramètre(s) d'intérêt et la configuration modèle CMk de milieu cible 2, donc le nombre de couches N et les paramètres de niveau 1, connaissant le signal photoacoustique ou photothermique simulé et les paramètres d'irradiation du cas d'irradiation, comme représenté sur la figure 7.

Le modèle appris, qu'on appellera modèle inverse par la suite, peut être transmis au module d'adaptation 14 et stocké en mémoire de ce module.

Plusieurs modèles inverses différents peuvent être appris, avec des jeux d'apprentissage et/ou des règles d'apprentissage différents.

## Abaque de fréquences nodales

On a vu précédemment que l'on peut générer, éventuellement de manière automatisée et/ou aléatoire, un grand nombre de configurations modèles CMk, chaque configuration modèle CMk correspondant à un nombre de couches N et un jeu de paramètres de niveau 1, et optionnellement à une valeur ou une gamme de valeurs du paramètre d'intérêt, décrivant une situation particulière du milieu stratifié cible 2 d'intérêt.

On peut donc générer des groupes GMp de configurations modèles, deux configurations modèles d'un même groupe ne différant que par la valeur du paramètre d'intérêt.

Pour chaque configuration modèle CMk, on a aussi vu que l'on peut générer, éventuellement de manière automatisée et/ou aléatoire un grand nombre de cas d'irradiations Ij, chaque cas d'irradiation correspondant à un jeu de paramètres d'irradiation décrivant les paramètres de la (des) sources de lumière 11a utilisée(s) pour l'irradiation.

On peut donc aussi générer des groupes GIq de cas d'irradiations, deux cas d'irradiation de chaque groupe GIq de cas d'irradiation ne différant que par la fréquence de modulation d'une source de lumière 11a.

Pour un groupe GMp de configurations modèles CMk qui ne différent les unes des autres que de par la valeur du paramètre d'intérêt, on peut générer les courbes d'amplitude du signal détecté en fonction de la fréquence de modulation de la source de lumière, au moyen d'irradiations simulées avec un groupe de cas d'irradiations GIq donné, seule la fréquence de modulation variant d'un cas d'irradiation à l'autre. C'est notamment ce qui a été fait pour obtenir les figures 3a et 3b.

A partir de ces courbes, éventuellement au moyen du processeur du module de simulation 15 ou du processeur du module d'adaptation 14, on peut déterminer au moins une fréquence de modulation nodale pour cet ensemble de configurations modèles GMp.

Il est donc possible de déterminer une pluralité de multiplet, appelé par la suite multiplet nodal. Chaque multiplet nodal comprend :

- les caractéristiques communes à tous les éléments d'un groupe GMp donné de configurations modèles du milieu stratifié cible (nombre de couches, épaisseurs des couches, concentrations ... à l'exclusion du paramètre d'intérêt qui varie d'un élément du groupe GMp à un autre ;
- et une pluralité de fréquences de modulation nodales associées à ce groupe GMp, pour lesquelles le signal acoustique ou thermique détecté par la cellule de détection en réponse à une irradiation par la source de lumière présente une corrélation inférieure à un seuil prédéterminé avec le paramètre d'intérêt.

A partir de la base de données de configurations modèles, on peut donc bien générer, par exemple au moyen du module de simulation 15, un abaque de fréquences nodales.

Une fois les simulations réalisées, on peut stocker dans une mémoire du module de simulation cet abaque de fréquences nodales, l'abaque comprenant au moins une pluralité de multiplets nodaux comprenant chacun au moins les caractéristiques communes (ou encore le jeu de paramètre d'irradiation communs) à un groupe de configurations modèles GMp et une pluralité de fréquences de modulation nodales associées.

Les multiplets nodaux peuvent optionnellement comprendre une ou plusieurs fréquences dites particulières, notées foptim, pour lesquelles la sensibilité du capteur est maximale dans la gamme de fréquences de modulation délimitée par deux fréquences de modulation nodales (éventuellement successives) pour le paramètre d'intérêt concerné.

Une telle fréquence de modulation particulière foptim peut être observée dans la zone 1 représentée sur la figure 3a.

Dans un mode de réalisation, une fréquence de modulation particulière est déterminée au moyen du processeur du module de simulation 15 ou du module d'adaptation 14 sur la base d'une moyenne pondérée d'au moins deux fréquences de modulation nodales successives.

A ce stade, les éléments nécessaires pour la mise en oeuvre du procédé de mesure suivant l'invention sont prêts.

On comprend d'ores et déjà que grâce à l'abaque de fréquences nodales, il va être possible de choisir les paramètres d'irradiation pour tenir compte des phénomènes d'interférences et ainsi réduire le nombre de fréquences de modulation d'irradiation nécessaires pour satisfaire un ou plusieurs critères d'optimisation, notamment parmi un critère de consommation énergétique, un critère de sensibilité, un critère de précision.

L'abaque de fréquences nodales peut être stocké dans une mémoire du module d'adaptation 14 ou bien du module de simulation 15 ou une autre mémoire du capteur non invasif 1.

En variante, l'abaque de fréquences nodales est généré à partir de données obtenues via des expérimentations in vivo. Dans ce mode de réalisation, le module de simulation 15 n'est pas nécessaire.

Dans un autre mode de réalisation, l'abaque de fréquences nodales est généré à la fois à partir de données obtenues in silico au moyen du module de simulation et de données obtenues in vivo.

## Procédé de mesure du paramètre d'intérêt

Un mode de réalisation particulier du procédé de mesure du paramètre d'intérêt au moyen du capteur non invasif 1 basé sur la détection photoacoustique indirecte ou photothermique est représenté sur la figure 5. Il comprend les étapes suivantes :

a) *Initialisation :*

Le module d'adaptation 14 choisit une configuration modèle initiale de milieu stratifié cible 2 en vue d'une irradiation sur la base de critères prédéterminés. Par exemple, pour la figure 5, la configuration modèle CMirrad initiale est la configuration modèle CMk.

Différentes options peuvent être retenues pour cette initialisation.

Notamment, dans le cas de la mesure de la glycémie interstitiel à partir de la base de données de configuration modèles et/ou d'une base de données de mesures expérimentales, on peut déterminer un jeu de paramètres de niveau 1 et de glycémie moyen pour une population de patients ou pour un patient donné. Ce jeu de paramètres moyen correspond à une configuration modèle initiale CMk du milieu stratifié cible 2 qui a la plus grande probabilité d'être la plus adaptée pour la mesure à venir en l'absence de toute autre information et notamment en l'absence d'un historique de mesure.

b) *Adaptation des paramètres d'irradiation*

1- A partir de l'abaque de fréquences nodales, le processeur du module d'adaptation 14 détermine une pluralité de fréquences de modulation nodales fnod associées au groupe GMp de configurations modèles du milieu stratifié cible auquel appartient la configuration modèle initiale CMirrad ;

2- Puis il détermine au moins une fréquence de modulation particulière foptim sur la base de cette pluralité de fréquences de modulation nodales. L'au moins une fréquence de modulation particulière foptim peut être une fréquence de modulation pour lesquelles la sensibilité du capteur est maximale dans la gamme de fréquences de modulation délimitée par deux fréquences de modulation nodales (éventuellement successives) pour le paramètre d'intérêt concerné. Dans un mode de réalisation, l'au moins une fréquence de modulation particulière foptim est déterminée au moyen du processeur du module de simulation 15 ou du module d'adaptation 14 sur la base d'une moyenne pondérée d'au moins deux fréquences de modulation nodales successives.

3- Et enfin, il détermine sur la base de ces fréquences de modulation nodales un cas d'irradiation particulier pour la configuration modèle du milieu stratifié cible 2 CMirrad, qui est un cas d'irradiation comprenant au moins la fréquence d'irradiation particulière foptim pour la configuration modèle initiale CMirrad. En particulier, le cas d'irradiation particulier peut être noté Iopt.k puisqu'il est optimisé par rapport à un critère de prise en compte des phénomènes d'interférences.

c) *Première irradiation :* On procède à une première irradiation du milieu cible 2, l'une au moins de la ou des sources de lumière 11a étant configurée suivant le jeu de paramètres d'irradiation correspondant au cas d'irradiation particulier.

d) *Détection PA :*

On détecte au moyen de la cellule de détection photoacoustique 12 le signal photoacoustique réel généré en réponse à l'irradiation.

Le cas échéant, le module de traitement du signal 13 reçoit et traite ce signal photoacoustique réel et le transmet après traitement au module d'adaptation 14.

e) *Détermination du paramètre d'intérêt :*

Le processeur du module d'adaptation 14 détermine le paramètre d'intérêt, dans cet exemple la glycémie, sur la base du signal photoacoustique ou photothermique détecté.

Plusieurs modes de réalisation de l'étape e) sont possibles.

Notamment, le processeur du module d'adaptation 14 peut implémenter un modèle inverse unique.

En variante, le processeur du module d'adaptation 14 peut utiliser une loi de fonctionnement permettant de déduire du signal détecté une valeur de glycémie.

Dans le mode de réalisation plus perfectionné, représenté sur la figure 5, l'étape e) peut se décomposer de la manière suivante :

e1) *Résolution du problème inverse* :

Le processeur du module d'adaptation 14 implémentant le(s) modèle(s) inverse appris (et notamment au moins celui correspondant à la configuration modèle CMk du milieu stratifié cible 2 utilisée pour l'irradiation) reçoit en entrée le signal photoacoustique ou photothermique réel et le cas d'irradiation particulier sélectionné pour l'irradiation, noté par exemple Iopt.k, et détermine la configuration modèle de milieu stratifié cible 2 en cours, noté CMmes, ainsi que le paramètre d'intérêt (comme représenté sur la figure 7).

e2) *Validation de la configuration modèle :*

Le module d'adaptation 14 compare la configuration modèle de milieu stratifié cible 2 en cours CMmes à la configuration modèle CMirrad utilisée pour l'irradiation (configuration modèle CMk issue de l'étape initialisation pour la première mesure, configuration modèle CMℓ éventuellement différente pour une mesure ultérieure).

Cas 1) Si la configuration modèle de milieu stratifié cible 2 mesurée CMmes est identique à la configuration modèle CMirrad qui a été utilisée pour l'irradiation, la pluralité de fréquences de modulation nodales utilisées pour déterminer la fréquence de modulation particulière foptim et en conséquence les paramètres d'irradiation choisis, c'est-à-dire le cas d'irradiation particulier, étaient adaptées à la situation physiologique en cours de mesure. Autrement dit, le cas d'irradiation particulier sélectionné était optimisé pour tenir compte des phénomènes d'interférences pour la situation physiologique en cours (qui on le rappelle n'est pas connue a priori et évolue au cours du temps), de même que le modèle inverse utilisé pour déterminer le paramètre d'intérêt. En conséquence, le paramètre d'intérêt déterminé à l'étape e1) par le module d'adaptation 14 est le résultat de la mesure.

Cas 2) Si la configuration modèle de milieu stratifié cible 2 mesurée CMmes est différente de la configuration modèle CMirrad, le processeur du module d'adaptation recherche alors dans l'abaque de fréquences nodales une nouvelle pluralité de fréquences de modulation nodales, correspondant cette fois au groupe de configurations modèles GMp' auquel appartient la nouvelle configuration modèle de milieu stratifié cible CMmes et il détermine, sur la base de cette pluralité de fréquences de modulation nodales, un nouveau cas d'irradiation particulier qui comprend au moins une fréquence de modulation foptim déterminée sur la base de la pluralité de fréquences de modulation nodales pour la configuration modèle de milieu stratifié cible 2 CMmes. Le nouveau cas d'irradiation particulier est par exemple noté Iopt. ℓ, dans la mesure où il est optimisé pour tenir compte des phénomènes d'interférences pour la situation physiologique mesurée. Le processeur du module d'adaptation 14 transmet les paramètres correspondants au dispositif d'irradiation 11.

Le milieu stratifié cible 2 subit alors une nouvelle irradiation (irradiation ultérieure) suivant les paramètres d'irradiation du cas d'irradiation Iopt. ℓ. Puis les étapes d) de détection et, le cas échéant de traitement du signal photoacoustique ou photothermique et e1) de résolution du problème inverse sont réitérées.

Le procédé peut comprendre au plus une étape e2) de validation de la configuration modèle.

En variante, le procédé peut comprendre aussi une réitération de l'étape e2) de validation de la configuration modèle.

Dans tous les cas, à l'étape e), la configuration modèle de milieu stratifié cible 2 en cours CMmes peut être différente de la configuration modèle CMirrad. En effet, CMirrad a été choisie

- soit suivant les critères de l'initialisation (CMirrad est alors la configuration modèle « moyenne », notamment en l'absence d'historique de mesure sur le patient. C'est le cas pour la première irradiation). ;
- soit sur la base de la mesure précédente (CMirrad est alors la configuration modèle la plus adaptée pour le patient connaissant le résultat de la mesure suite à l'irradiation précédente).

La validation de CMirrad se fait donc grâce à une information supplémentaire acquise grâce à l'irradiation en cours, à savoir le signal détecté par la cellule de détection photoacoustique 12. Si par hasard, CMmes = CMirrad, il n'est pas nécessaire de procéder à une nouvelle irradiation et la valeur du paramètre d'intérêt est bien la plus précise qui pouvait être obtenue, mais l'étape de validation de la configuration modèle a apporté une information supplémentaire à savoir la confirmation que la précision de la mesure est bien maximale pour ce cas.

Dans le cas, CMmes est différent de CMirrad, l'adaptation des paramètres d'irradiation et de la configuration modèle permet d'accroître la précision de la mesure au prix d'au moins une irradiation supplémentaire mais avec une consommation énergétique toujours maîtrisée, et grâce à la seconde étape de validation, de confirmer que la précision de la mesure est bien maximale. Dans le cas où l'on autorise la réitération de l'étape de validation (comme représenté sur la figure 5), on acquiert en sortie du procédé l'information supplémentaire que la précision de la mesure est bien maximale.

En général, dans ce cas, le résultat de mesure du paramètre d'intérêt est obtenu après la première irradiation ou après deux irradiations. Cependant, pour s'assurer que le procédé converge et/ou limiter la consommation énergétique, on peut prévoir une limitation du nombre d'étapes de validation de la configuration modèle.

Dans tous les cas, on constate donc que le choix d'un cas d'irradiation sur la base de l'abaque de fréquences nodales permet de limiter le nombre de fréquences de modulation et de longueurs d'onde utilisées pour l'irradiation en ne conservant que les valeurs porteuses d'informations non redondantes sur la configuration modèle de milieu cible 2 physiologique en cours et suffisantes pour obtenir la précision de mesure souhaitée et/ou optimales pour limiter la consommation en énergie du capteur à une valeur prédéterminée.

Notamment, la ou les fréquences de modulation particulières des sources sont sélectionnées pour tenir compte des phénomènes d'interférences : une fréquence de modulation particulière foptim est une fréquence de modulation pour laquelle la sensibilité de la mesure est la plus grande entre deux fréquences de modulation nodales. Le choix d'un cas d'irradiation comprenant au moins une fréquence de modulation particulière permet d'accroître la sensibilité du capteur sans pour autant accroître sa consommation énergétique.

On pourra s'en convaincre en observant les figures 8a, 8b et 8c : pour obtenir ces trois figures, trois situations successives ont été simulées. Pour les trois figures, le milieu stratifié cible 2 est la peau d'un patient, modélisée par un modèle bicouche (stratum corneum, épiderme). L'épaisseur de la couche supérieure (modélisant le stratum corneum) est égale à 14 $\mu$m pour la figure 8a, et on suppose qu'un événement fait varier cette épaisseur de sorte qu'elle évolue et devienne égale à 18 μm lorsque les mesures pour la figure 8b et la figure 8c sont réalisées. L'épaisseur de la couche inférieure (modélisant l'épiderme) est considérée comme infinie. La glycémie est nulle dans la couche supérieure. La couche supérieure comprend 20% d'eau et la couche inférieure comprend 60% d'eau.

L'abaque de fréquences nodales permet de déterminer, au moyen du processeur du module d'adaptation 14, une pluralité de fréquences nodales pour la situation initiale (épaisseur du stratum corneum égale à 14 μm) et de déterminer le cas d'irradiation à utiliser pour cette situation sur la base de ces fréquences nodales. En l'occurrence, pour l'obtention des points de mesures de la figure 8a, le modèle de peau est irradié (de manière simulée) avec un laser de nombre d'onde 1034 cm$^{-1}$ modulé en intensité aux trois fréquences de modulation foptim = 1580 Hz, fnod1 = 950 Hz et fnod2 = 4750 Hz déterminées à partir de la figure 3a.

De même, l'abaque de fréquences nodales permet de déterminer, au moyen du processeur du module d'adaptation 14, une pluralité de fréquences de modulation nodales pour la situation finale (épaisseur du stratum corneum égale à 18 μm) et de déterminer le cas d'irradiation à utiliser pour cette situation sur la base de ces fréquences nodales. En l'occurrence, pour l'obtention des points de mesure de la figure 8c, le modèle de peau est irradié (de manière simulée) avec un laser de nombre d'onde 1034 cm$^{-1}$ modulé en intensité aux trois fréquences de modulation foptim = 1000 Hz, f'nod1 = 600 Hz et f'nod2 = 3000 Hz déterminées à partir de la figure 3b.

En revanche, pour l'obtention des points de mesure de la figure 8b, le modèle de peau est irradié avec un laser de nombre d'onde 1034 cm$^{-1}$ modulé en intensité aux trois fréquences de modulation particulières adaptées au modèle de peau de la figure 8a, à savoir foptim = 1580 Hz, fnod1 = 950 Hz et fnod2 = 4750 Hz.

On constate que dans le cas où le cas d'irradiation est choisie sur la base de fréquences de modulation nodales adaptées à la situation physiologique en cours, c'est-à-dire pour les figures 8a et 8c, l'écart quadratique moyen (RMSE) entre les valeurs prédites et réelles est acceptable (respectivement 18,5 mg/dL et 15,4 mg/dL). Par contre, si les fréquences de modulation nodales sont inadaptées (cas de la figure 8b), l'écart quadratique moyen (RMSE) entre les valeurs prédites et réelles se dégrade fortement et atteint dans ce cas 28,4 mg/dL.

Il apparaît donc clairement que, si le choix d'un nombre restreint de fréquences de modulation sur la base des fréquences de modulation nodales permet de contrôler la consommation du capteur non invasif, l'adaptation des fréquences de modulation nodales (sur la base desquelles sont déterminées les fréquences de modulation particulières) à la situation physiologique en cours permet de contrôler en outre la précision du capteur non invasif 1, par exemple de manière à ce que l'écart quadratique moyen ou tout autre grandeur permettant de quantifier la précision soit inférieure à un seuil prédéterminé.

Le procédé de détection peut comprendre la sélection pour chaque cas d'irradiation d'une seule fréquence de modulation particulière foptim déterminée sur la base d'une pluralité de fréquences nodales, par exemple au moyen d'une moyenne arithmétique ou pondérée. Notamment, dans le cas d'une moyenne pondérée, un poids plus important peut être attribué à la fréquence de modulation nodale la plus faible.

Cette disposition permet de tenir compte du fait que l'amplitude du signal photothermique décroît avec la fréquence de modulation de l'intensité de l'onde lumineuse incidente.

A titre d'exemple, dans le cas particulier d'une mesure photothermique dans le cas du régime du piston thermique décrit dans Kottmann, J. et al. (2012) ; Glucose sensing in human epidermis using mid-infrared photoacoustic détection; Biomedical optics express, 3(4), 667-680, l'amplitude du signal photothermique est proportionnelle à l'inverse de la fréquence de modulation de l'onde lumineuse incidente. En conséquence, notamment dans ce cas, une fréquence de modulation particulière foptim peut être le barycentre de deux fréquences de modulation nodales affectées chacune d'un poids égal à son inverse. Si on note f1 et f2 deux fréquences de modulation nodales successives, la fréquence de modulation particulière peut donc être calculée de la manière suivante :

Formule 2

$$foptim = \frac{\frac{1}{f1}f1 + \frac{1}{f2}f2}{\frac{1}{f1} + \frac{1}{f2}} = 2\frac{f1f2}{f1 + f2}$$

Dans un autre mode de réalisation, adapté notamment au cas d'une cellule de détection photoacoustique fermée, pour tenir compte de la réponse fréquentielle de la cellule de détection, une fréquence de modulation particulière foptim peut être le barycentre de deux fréquences de modulation nodales successives affectées chacune d'un poids égal à son inverse élevé à la puissance 3/2.

D'autres poids peuvent être encore choisis, par exemple pour tenir compte des réponses fréquentielles d'un ou plusieurs autres éléments de la chaîne de mesure.

En variante, pour chaque irradiation, le cas d'irradiation sélectionné dans le procédé peut comprendre, en plus d'une fréquence de modulation particulière foptim, au moins une fréquence de modulation nodale. En effet, si le signal détecté correspondant à la fréquence de modulation nodale n'est pas porteur d'information concernant le paramètre d'intérêt, il est en revanche porteur d'information concernant la structuration du milieu stratifié 2 indépendamment de ce paramètre d'intérêt. La sélection à la fois d'une fréquence de modulation particulière foptim et d'une fréquence de modulation nodale fnod peut donc conduire à la résolution du problème inverse avec une efficacité et une précision accrues par rapport à toute autre sélection de deux fréquences de modulation.

En particulier, comme cela a été fait pour les exemples des figures 8a, 8b et 8c, il est possible de sélectionner une ou les deux fréquences de modulation nodales les plus proches de la fréquence optimale foptim déterminée.

Il est aussi possible de sélectionner deux ou plus de fréquences de modulation particulières différentes.

Par exemple, si le principal critère d'optimisation est la consommation énergétique, par exemple si le capteur non invasif est alimenté par une batterie qui atteint un seuil inférieur prédéterminé de charge, il est possible de ne sélectionner pour l'irradiation qu'une seule fréquence de modulation, à savoir une fréquence de modulation particulière foptim.

Si le critère de consommation énergétique est moins important et qu'une irradiation suivant deux fréquences de modulation différente est permise, on peut utiliser pour l'irradiation une fréquence de modulation particulière foptim et la fréquence de modulation nodale la plus faible parmi celles utilisées pour déterminer foptim. En effet, c'est à cette fréquence de modulation nodale la plus faible que l'amplitude du signal sera la plus élevée et donc la précision la meilleure amplitude.

Si on cherche à optimiser la sensibilité ou la précision du capteur avec une consommation énergétique encore moins limitée, on peut utiliser pour l'irradiation une fréquence de modulation particulière foptim et deux fréquences de modulation nodale pour déterminer foptim, et optionnellement en outre une deuxième, une troisième, etc, fréquence de modulation particulière.

L'invention porte enfin sur un programme d'ordinateur comprenant des instructions qui conduisent le capteur non invasif 1 suivant l'une des modes de réalisation précédents à exécuter les étapes du procédé suivant l'un quelconque des modes de réalisation décrits plus hauts.

## LISTE DES SIGNES DE RÉFÉRENCE

1 : capteur non invasif basé sur la détection photoacoustique indirecte
11 : dispositif d'irradiation
11a: source de lumière
11b : dispositif de modulation de l'intensité de la source de lumière 11a
11c : dispositif de contrôle de la fréquence de modulation fmod de l'intensité de la source de lumière 11a
12 : cellule de détection
13 : module de traitement du signal
14 : module d'adaptation
15 : module de simulation
2 : milieu stratifié cible
2A : première couche du milieu stratifié cible
2B : deuxième couche du milieu stratifié cible

**Revendications**

1.  Procédé de mesure d'un paramètre d'intérêt dans un milieu stratifié cible (2) au moyen d'un capteur non invasif (1) basé sur la détection photoacoustique ou la détection photothermique, comprenant :

    a) on fournit un capteur comprenant :

        - une source de lumière (11a),
        - un dispositif de contrôle d'une pluralité de paramètres d'irradiation de la source de lumière (11a), la pluralité de paramètres d'irradiation comprenant au moins une fréquence de modulation de l'intensité de la source de lumière,
        - une cellule de détection configurée pour détecter un signal acoustique ou thermique (12),
        - une mémoire dans laquelle est stocké un abaque de fréquences nodales comprenant, pour :

            * une pluralité de groupes (GMp) de configurations modèles du milieu stratifié cible, comprenant chacun au moins deux configurations modèles (CMk) du milieu stratifié cible ne différant les unes des autres que par le paramètre d'intérêt,

\* et une pluralité de groupes (GIq) de cas d'irradiation comprenant chacun au moins deux cas d'irradiation (Ij) ne différant les uns des autres que par une fréquence de modulation d'une source de lumière, chaque cas d'irradiation (Ij) comprenant un jeu de valeurs de paramètres d'irradiation, une pluralité de multiplets nodaux, chaque multiplet nodal comprenant des caractéristiques communes à tous les éléments d'un groupe (GMp) donné de configurations modèles du milieu stratifié cible et une pluralité de fréquences de modulation nodales associées, pour lesquelles le signal acoustique ou thermique détecté par la cellule de détection en réponse à une irradiation par la source de lumière présente une corrélation inférieure à un seuil prédéterminé avec le paramètre d'intérêt ;

- et un module d'adaptation (14) comprenant un processeur et échangeant des informations avec la cellule de détection (12) et le dispositif de contrôle des paramètres d'irradiation de la source de lumière ;

b) le module d'adaptation (14) choisit une configuration modèle du milieu stratifié cible (CMirrad) initiale en vue d'une irradiation ;

c) le processeur du module d'adaptation (14) :

- détermine dans l'abaque de fréquences nodales une pluralité de fréquences de modulation nodales (fnod) associées à un groupe (GMp) de configurations modèles du milieu stratifié cible auquel appartient la configuration modèle du milieu stratifié choisie (CMirrad),
- puis détermine au moins une fréquence de modulation particulière (foptim) sur la base de ladite pluralité de fréquences de modulation nodales, ladite fréquence de modulation particulière étant une moyenne pondérée d'au moins deux de ladite pluralité de fréquences de modulation nodales et étant délimitée par deux fréquences de modulation nodales ,
- et détermine un cas d'irradiation particulier pour la configuration modèle du milieu stratifié cible choisie (CMirrad), le cas d'irradiation particulier comprenant l'au moins une fréquence de modulation particulière (foptim);

d) la source de lumière (11a) irradie le milieu stratifié cible (2) suivant le jeu de paramètres d'irradiation dudit cas d'irradiation particulier ;
e) la cellule de détection (12) détecte un signal acoustique ou thermique généré en réponse à l'irradiation ;
f) le processeur du module d'adaptation (14) détermine le paramètre d'intérêt sur la base du signal acoustique ou thermique détecté.

2. Procédé de mesure suivant la revendication 1 dans lequel :

- le processeur du module d'adaptation (14) implémente de plus un algorithme de modélisation inverse recevant en entrée un cas d'irradiation (Ij) et un signal acoustique ou thermique et fournissant en sortie une configuration modèle (CMk) du milieu stratifié cible et une valeur du paramètre d'intérêt,
- l'étape f) comprend les étapes suivantes :

f1) le processeur du module d'adaptation reçoit en entrée le signal acoustique ou thermique détecté par la cellule de détection (12) et le cas d'irradiation particulier utilisé pour l'irradiation, et renvoie en sortie de l'algorithme de modélisation inverse une configuration modèle du milieu stratifié cible en cours (CMmes) et une valeur du paramètre d'intérêt estimée (Pest) ;
f2) le processeur du module d'adaptation (14) évalue la configuration modèle du milieu stratifié cible choisie (CMirrad) par comparaison avec la configuration modèle du milieu stratifié cible en cours (CMmes), et, seulement si CMirrad est différent de CMmes : g) le module d'adaptation (14) reçoit en entrée la configuration modèle du milieu stratifié cible en cours (CMmes) et renvoie en sortie une nouvelle configuration modèle du milieu stratifié cible choisie en vue d'une irradiation (CMirrad), puis on réitère c), d), e) et f1) ;
f3) la valeur du paramètre d'intérêt mesurée (Pmes) par le capteur est la dernière valeur du paramètre d'intérêt estimée (Pest).

3. Procédé de mesure suivant la revendication 1, g) comprenant en outre on réitère f2) à l'issue de f1).

4. Procédé de mesure suivant l'une des revendications 1 à 3 comprenant au préalable :

I- au moyen d'un processeur et d'une base de données de configurations modèles comprenant des multiplets (configuration modèle (CMk) du milieu stratifié cible, cas d'irradiation (Ij), paramètre d'intérêt) et un signal acoustique ou thermique détecté par la cellule de détection (12) associé à chacun desdits multiplets, on génère un abaque de fréquences nodales pour :

- une pluralité de groupes (GMp) de configurations modèles,
- et une pluralité de groupes (GIq) de cas d'irradiation,
l'abaque de fréquences nodales comprenant une pluralité de multiplets nodaux, chaque multiplet nodal comprenant des caractéristiques communes à tous les éléments d'un groupe (GMp) de configurations modèles du milieu stratifié cible et une pluralité de fréquences de modulation nodales associées, et on stocke cet abaque de fréquences nodales dans la mémoire du capteur non invasif (1).

5. Procédé de mesure suivant la revendication 4 comprenant :
II- un processeur apprend au moins un algorithme de modélisation inverse à partir de la base de données de configurations modèles et on stocke l'au moins un algorithme de modélisation inverse dans la mémoire du capteur non invasif (1).

6. Procédé de mesure suivant l'une quelconque des revendications 4 à 5 dans lequel au moins une partie des signaux acoustiques ou thermiques détectés par la cellule de détection (12) associés aux multiplets stockés dans la base de données de configurations modèles sont simulés, c'est-à-dire qu'ils sont générés au moyen d'un dispositif informatisé de simulation.

7. Procédé de mesure suivant l'une quelconque des revendications 1 à 6 dans lequel ledit cas d'irradiation particulier pour la configuration modèle choisie déterminé par le module d'adaptation (14) à l'étape c) comprend en outre au moins une fréquence de modulation nodale.

8. Capteur non invasif (1) basé sur la détection photoacoustique ou photothermique configuré pour mesurer un paramètre d'intérêt dans un milieu stratifié cible (2) comprenant :

- une source de lumière (11a),
- un dispositif de contrôle des paramètres d'irradiation de la source de lumière (1 1a),
- une cellule de détection (12) configurée pour détecter un signal acoustique ou thermique,
- une mémoire dans laquelle est stockée un abaque de fréquences nodales comprenant, pour :
- une pluralité de groupes (GMp) de configurations modèles du milieu stratifié cible, comprenant chacun au moins deux configurations modèles (CMk) du milieu stratifié cible ne différant les unes des autres que par le paramètre d'intérêt,
- et une pluralité de groupes (GIq) de cas d'irradiation comprenant chacun au moins deux cas d'irradiation (Ij) ne différant les uns des autres que par une fréquence de modulation d'une source de lumière, chaque cas d'irradiation (Ij) comprenant un jeu de valeurs de paramètres d'irradiation, une pluralité de multiplets dits « nodaux », chaque multiplet comprenant des caractéristiques communes à tous les éléments d'un groupe (GMp) donné de configurations modèles du milieu stratifié cible et une pluralité de fréquences de modulation nodales associées, pour lesquelles le signal acoustique ou thermique détecté par la cellule de détection en réponse à une irradiation par la source de lumière présente une corrélation inférieure à un seuil prédéterminé avec le paramètre d'intérêt,
le capteur non invasif comprenant en outre un module d'adaptation (14) comprenant un processeur et adapté pour échanger des informations avec la cellule de détection (12) et le dispositif de contrôle des paramètres d'irradiation de la source de lumière (11a),
le module d'adaptation (14) étant en outre configuré pour :

- choisir une configuration modèle du milieu stratifié cible (CMirrad) initiale,
- déterminer dans l'abaque de fréquences nodales une pluralité de fréquences de modulation nodales (fnod) associées à un groupe (GMp) de configurations modèles auquel appartient la configuration modèle du milieu stratifié cible choisie (CMirrad),
- déterminer au moins une fréquence de modulation particulière (foptim) sur la base de ladite pluralité de fréquences de modulation nodales, ladite fréquence de modulation particulière étant une moyenne pondérée d'au moins deux de ladite pluralité de fréquences de modulation nodales et étant délimitée par deux fréquences de modulation nodales,
- déterminer un cas d'irradiation particulier pour la configuration modèle d'irradiation choisie (CMirrad) comprenant l'au moins une fréquence de modulation particulière (foptim) ;
- transmettre un cas d'irradiation particulier au dispositif de contrôle des paramètres d'irradiation de la source de lumière,
- recevoir un signal acoustique ou thermique détecté par la cellule de détection (12),
- déterminer la valeur du paramètre d'intérêt sur la base du signal acoustique ou thermique détecté.

9. Capteur non invasif suivant la revendication 8 dans lequel le processeur du module d'adaptation est de plus adapté pour :

- implémenter un algorithme de modélisation inverse recevant en entrée un cas d'irradiation (Ij) comprenant un jeu de paramètres d'irradiation et un signal acoustique ou thermique et fournissant en sortie une configuration modèle (CMk) du milieu stratifié cible et une valeur du paramètre d'intérêt ;
- déterminer au moyen de l'algorithme de modélisation inverse une configuration modèle du milieu stratifié cible en cours (CMmes) et une valeur du paramètre d'intérêt estimée (Pest) sur la base d'un signal photoacoustique ou photothermique détecté reçu et d'un cas d'irradiation choisi ;
- évaluer une configuration modèle d'irradiation du milieu stratifié cible choisie (CMirrad) par comparaison avec une configuration modèle du milieu stratifié cible en cours (CMmes) ;
- seulement si la configuration modèle d'irradiation du milieu stratifié cible choisie (CMirrad) et la configuration modèle du milieu stratifié cible en cours (CMmes) comparées sont différentes :

    *déterminer une nouvelle configuration modèle du milieu stratifié cible choisie en vue d'une irradiation (CMirrad) lorsqu'il reçoit une configuration modèle du milieu stratifié cible en cours (CMmes),
    * déterminer dans l'abaque de fréquences nodales une pluralité de fréquences de modulation nodales (fnod) associées à un groupe (GMp) de configurations modèles du milieu stratifié cible auquel appartient la nouvelle configuration modèle du milieu stratifié choisie (CMirrad),
    * puis déterminer au moins une fréquence de modulation particulière (foptim) sur la base de ladite pluralité de fréquences de modulation nodales, ladite fréquence de modulation particulière étant une moyenne pondérée d'au moins deux de ladite pluralité de fréquences de modulation nodales et étant délimitée par deux fréquences de modulation nodales,
    * déterminer un nouveau cas d'irradiation particulier pour la nouvelle configuration modèle du milieu stratifié cible choisie (CMirrad), le cas d'irradiation particulier comprenant l'au moins une fréquence de modulation particulière (foptim),
    * et transmettre le nouveau cas d'irradiation particulier au dispositif de contrôle des paramètres d'irradiation de la source de lumière (11a) de sorte que la source de lumière (11a) irradie le milieu stratifié cible (2) suivant le jeu de paramètres d'irradiation dudit nouveau cas d'irradiation particulier, que la cellule de détection (12) détecte un signal acoustique ou thermique généré en réponse à cette nouvelle irradiation et le processeur du module d'adaptation (14) détermine le paramètre d'intérêt sur la base du nouveau signal acoustique ou thermique détecté;

- déterminer la valeur du paramètre d'intérêt mesurée (Pmes) sur la base de la dernière valeur du paramètre d'intérêt estimée (Pest).

10. Capteur non invasif suivant l'une quelconque des revendications 8 et 9 dans lequel au moins deux des éléments parmi la source de lumière (1 1a), le dispositif de contrôle des paramètres d'irradiation de la source de lumière (11a), la cellule de détection (12) configurée pour détecter un signal acoustique ou thermique, la mémoire dans laquelle est stocké un abaque de fréquences nodales et le module d'adaptation (14) sont indépendants mécaniquement l'un de l'autre.

11. Programme d'ordinateur comprenant des instructions qui conduisent le capteur non invasif (1) suivant l'une des revendication 8 à 10 à exécuter les étapes du procédé suivant l'une quelconque des revendications 1 à 7.

**Patentansprüche**

1. Verfahren zum Messen eines Parameters von Interesse in einem geschichteten Zielmedium (2) mittels eines nichtinvasiven Sensors (1), der auf photoakustischer Detektion oder photothermischer Detektion basiert, das Folgendes beinhaltet:

a) Bereitstellen eines Sensors, der Folgendes umfasst:

- eine Lichtquelle (11a),
- eine Vorrichtung zur Steuerung mehrerer Strahlungsparameter der Lichtquelle (11a), wobei die mehreren Strahlungsparameter mindestens eine Modulationsfrequenz der Intensität der Lichtquelle umfassen,
- eine Detektionszelle, die zum Detektieren eines akustischen oder thermischen Signals (12) konfiguriert ist,
- einen Speicher, in dem ein Knotenfrequenzdiagramm gespeichert ist, das für:

* mehrere Gruppen (GMp) von Modellkonfigurationen des geschichteten Zielmediums, von denen jede mindestens zwei Modellkonfigurationen (CMk) des geschichteten Zielmediums umfasst, die sich nur durch den Parameter von Interesse voneinander unterscheiden,
* und mehrere Gruppen (GIq) von Strahlungsfällen, von denen jede mindestens zwei Strahlungsfälle (Ij) umfasst, die sich nur durch eine Modulationsfrequenz einer Lichtquelle voneinander unterscheiden, wobei jeder Strahlungsfall (Ij) einen Satz von Strahlungsparameterwerten umfasst,

mehrere Knotenbytes umfasst, wobei jedes Knotenbyte Charakteristiken umfasst, die allen Elementen einer gegebenen Gruppe (GMp) von Modellkonfigurationen des geschichteten Zielmediums gemeinsam sind, und mehrere assoziierte Knotenmodulationsfrequenzen, bei denen das von der Detektionszelle als Reaktion auf eine Strahlung durch die Lichtquelle detektierte akustische oder thermische Signal eine Korrelation unterhalb eines vorbestimmten Schwellenwerts mit dem Parameter von Interesse aufweist;
- und ein Anpassungsmodul (14), das einen Prozessor umfasst und Informationen mit der Detektionszelle (12) und der Vorrichtung zur Steuerung der Strahlungsparameter der Lichtquelle austauscht;

b) Auswählen, durch das Anpassungsmodul (14), einer ursprünglichen Modellkonfiguration des geschichteten Zielmediums (CMirrad) im Hinblick auf eine Strahlung;
c) durch den Prozessor des Anpassungsmoduls (14):

- Bestimmen, in dem Knotenfrequenzdiagramm, mehrerer Knotenmodulationsfrequenzen (fnod), die mit einer Gruppe (GMp) von Modellkonfigurationen des geschichteten Zielmediums assoziiert sind, zu der die ausgewählte Modellkonfiguration des geschichteten Mediums (CMirrad) gehört,
- dann Bestimmen mindestens einer besonderen Modulationsfrequenz (foptim) auf der Basis der genannten mehreren Knotenmodulationsfrequenzen, wobei die genannte besondere Modulationsfrequenz ein gewichteter Durchschnitt von mindestens zwei der genannten mehreren Knotenmodulationsfrequenzen ist und durch zwei Knotenmodulationsfrequenzen begrenzt wird,
- und Bestimmen eines besonderen Strahlungsfalls für die gewählte Modellkonfiguration des geschichteten Zielmediums (CMirrad), wobei der besondere Strahlungsfall die mindestens eine besondere Modulationsfrequenz (foptim) umfasst;

d) Bestrahlen, durch die Lichtquelle (11a), des geschichteten Zielmediums (2) gemäß dem Satz von Strahlungsparametern des genannten besonderen Strahlungsfalls;
e) Detektieren, durch die Detektionszelle (12), eines akustischen oder thermischen Signals, das als Reaktion auf die Strahlung erzeugt wird;
f) Bestimmen, durch den Prozessor des Anpassungsmoduls (14), des Parameters von Interesse auf der Basis des detektierten akustischen oder thermischen Signals.

**2.** Messverfahren nach Anspruch 1, wobei:

- der Prozessor des Anpassungsmoduls (14) zusätzlich einen inversen Modellierungsalgorithmus implementiert, der als Eingabe einen Strahlungsfall (Ij) und ein akustisches oder thermisches Signal empfängt und als Ausgabe eine Modellkonfiguration (CMk) des geschichteten Zielmediums und einen Wert des Parameters von Interesse liefert,
- Schritt f) die folgenden Schritte beinhaltet:

f1) durch den Prozessor des Anpassungsmoduls, Empfangen, als Eingabe, des von der Detektionszelle (12) erfassten akustischen oder thermischen Signals und des für die Strahlung verwendeten besonderen Strahlungsfalls, und Zurückgeben, als Ausgabe des inversen Modellierungsalgorithmus, einer aktuellen Modellkonfiguration des geschichteten Zielmediums (CMmes) und eines geschätzten Wertes des Parameters von Interesse (Pest);

f2) Bewerten, durch den Prozessor des Anpassungsmoduls (14), der ausgewählten Modellkonfiguration des geschichteten Zielmediums (CMirrad) durch Vergleichen mit der aktuellen Modellkonfiguration des geschichteten Zielmediums (CMmes) und nur dann, wenn CMirrad von CMmes verschieden ist: g) durch das Anpassungsmodul (14), Empfangen, als Eingabe, der aktuellen Modellkonfiguration des geschichteten Zielmediums (CMmes), und Zurückgeben, als Ausgabe, einer neuen ausgewählten Modellkonfiguration des geschichteten Zielmediums im Hinblick auf eine Strahlung (CMirrad), woraufhin c), d), e) und f1) wiederholt werden;

f3) wobei der vom Sensor gemessene Wert des Parameters von Interesse (Pmes) der letzte geschätzte Wert des Parameters von Interesse (Pest) ist.

3. Messverfahren nach Anspruch 1, wobei g) außerdem das Wiederholen von f2) nach Abschluss von f1) beinhaltet.

4. Messverfahren nach einem der Ansprüche 1 bis 3, das zuvor Folgendes beinhaltet:

I- Erzeugen, mittels eines Prozessors und einer Datenbank von Modellkonfigurationen, die Bytes (Modellkonfiguration (CMk) des geschichteten Zielmediums, Strahlungsfall (Ij), Parameter von Interesse) und ein akustisches oder thermisches Signal umfasst, das von der mit jedem der genannten Bytes assoziierten Detektionszelle (12) detektiert wird, einer Knotenfrequenzdiagramm für:

- mehrere Gruppen (GMp) von Modellkonfigurationen,
- und mehrere Gruppen (GIq) von Strahlungsfällen,

wobei das Knotenfrequenzdiagramm mehrere Knotenbytes umfasst, wobei jedes Knotenbyte allen Elementen einer Gruppe (GMp) von Modellkonfigurationen des geschichteten Zielmediums gemeinsame Charakteristiken und mehrere assoziierte Knotenmodulationsfrequenzen umfasst, und Speichern dieses Knotenfrequenzdiagramms im Speicher des nichtinvasiven Sensors (1).

5. Messverfahren nach Anspruch 4, das Folgendes beinhaltet:

II- Lernen, durch einen Prozessor, mindestens eines inversen Modellierungsalgorithmus auf der Basis der Modellkonfigurationsdatenbank und Speichern des mindestens einen inversen Modellierungsalgorithmus im Speicher des nichtinvasiven Sensors (1).

6. Messverfahren nach einem der Ansprüche 4 bis 5, wobei mindestens ein Teil der von der Detektionszelle (12) detektierten akustischen oder thermischen Signale, die mit den in der Modellkonfigurationsdatenbank gespeicherten Bytes assoziiert sind, simuliert wird, d.h. sie werden mithilfe einer computergestützten Simulationsvorrichtung erzeugt.

7. Messverfahren nach einem der Ansprüche 1 bis 6, wobei der genannte durch das Anpassungsmodul (14) in Schritt c) bestimmte besondere Strahlungsfall für die gewählte Modellkonfiguration zusätzlich mindestens eine Knotenmodulationsfrequenz umfasst.

8. Nichtinvasiver Sensor (1), der auf photoakustischer oder photothermischer Detektion basiert und zum Messen eines Parameters von Interesse in einem geschichteten Zielmedium (2) konfiguriert ist, der Folgendes umfasst:

- eine Lichtquelle (11a),
- eine Vorrichtung zur Steuerung der Strahlungsparameter der Lichtquelle (11a),
- eine Detektionszelle (12), die zum Detektieren eines akustischen oder thermischen Signals konfiguriert ist,
- einen Speicher, in dem ein Knotenfrequenzdiagramm gespeichert ist, das für:
- mehrere Gruppen (GMp) von Modellkonfigurationen des geschichteten Zielmediums, von denen jede mindestens zwei Modellkonfigurationen (CMk) des geschichteten Zielmediums umfasst, die sich nur durch den Parameter von Interesse voneinander unterscheiden,
- und mehrere Gruppen (GIq) von Strahlungsfällen, von denen jede mindestens zwei Strahlungsfälle (Ij) umfasst, die sich nur durch eine Modulationsfrequenz einer Lichtquelle voneinander unterscheiden, wobei jeder Strahlungsfall (Ij) einen Satz von Strahlungsparameterwerten umfasst,

mehrere sogenannte "Knoten"-Bytes umfasst, wobei jedes Byte allen Elementen einer gegebenen Gruppe (GMp) von Modellkonfigurationen des geschichteten Zielmediums gemeinsame Charakteristiken und mehrere assoziierte Knotenmodulationsfrequenzen umfasst, bei denen das von der Detektionszelle als Reaktion auf eine Strahlung durch die Lichtquelle detektierte akustische oder thermische Signal eine Korrelation unterhalb eines vorbestimmten Schwellenwerts mit dem Parameter
von Interesse aufweist,
wobei der nichtinvasive Sensor ferner ein Anpassungsmodul (14) umfasst, das einen Prozessor umfasst und zum Austauschen von Informationen mit der Detektionszelle (12) und der Vorrichtung zur Steuerung der Strahlungsparameter der Lichtquelle (11a) ausgelegt ist,
wobei das Anpassungsmodul (14) außerdem konfiguriert ist zum:

- Auswählen einer ursprünglichen Modellkonfiguration des geschichteten Zielmediums (CMirrad),
- Bestimmen, in dem Knotenfrequenzdiagramm, mehrerer Knotenmodulationsfrequenzen (fnod), die mit einer Gruppe (GMp) von Modellkonfigurationen assoziiert sind, zu der die ausgewählte Modellkonfiguration des geschichteten Zielmediums (CMirrad) gehört,
- Bestimmen mindestens einer besonderen Modulationsfrequenz (foptim) auf der Basis der genannten mehreren Knotenmodulationsfrequenzen, wobei die genannte besondere Modulationsfrequenz ein gewichteter Durchschnitt von mindestens zwei der genannten mehreren Knotenmodulationsfrequenzen ist und durch zwei Knotenmodulationsfrequenzen begrenzt wird,
- Bestimmen eines besonderen Strahlungsfalls für die ausgewählte Strahlungsmodellkonfiguration (CMirrad), die die mindestens eine besondere Modulationsfrequenz (foptim) umfasst;
- Senden eines besonderen Strahlungsfalls zu der Vorrichtung zur Steuerung der Strahlungsparameter der Lichtquelle,
- Empfangen eines akustischen oder thermischen Signals, das von der Detektionszelle (12) detektiert wurde,
- Bestimmen des Wertes des Parameters von Interesse auf der Basis des detektierten akustischen oder thermischen Signals.

9.  Nichtinvasiver Sensor nach Anspruch 8, wobei der Prozessor des Anpassungsmoduls zusätzlich ausgelegt ist zum:

- Implementieren eines inversen Modellierungsalgorithmus, der als Eingabe einen Strahlungsfall (Ij), der einen Satz von Strahlungsparametern umfasst, und ein akustisches oder thermisches Signal empfängt und als Ausgabe eine Modellkonfiguration (CMk) des geschichteten Zielmediums und einen Wert des Parameters von Interesse liefert;
- Bestimmen, mittels des inversen Modellierungsalgorithmus, einer aktuellen Modellkonfiguration des geschichteten Zielmediums (CMmes) und eines geschätzten Wertes des Parameters von Interesse (Pest) auf der Basis eines empfangenen detektierten photoakustischen oder photothermischen Signals und eines ausgewählten Strahlungsfalls;
- Bewerten einer ausgewählten Strahlungsmodellkonfiguration des geschichteten Zielmediums (CMirrad) durch Vergleichen mit einer aktuellen Modellkonfiguration des geschichteten Zielmediums (CMmes);
- nur dann, wenn die gewählte Strahlungsmodellkonfiguration des geschichteten Zielmediums (CMirrad) und die aktuelle Modellkonfiguration des geschichteten Zielmediums (CMmes), die verglichen wurden, unterschiedlich sind:

  * Bestimmen einer neuen ausgewählten Modellkonfiguration des geschichteten Zielmediums im Hinblick auf eine Strahlung (CMirrad), wenn er eine aktuelle Modellkonfiguration des geschichteten Zielmediums (CMmes) empfängt,
  * Bestimmen, in dem Knotenfrequenzdiagramm, mehrerer Knotenmodulationsfrequenzen (fnod), die mit einer Gruppe (GMp) von Modellkonfigurationen des geschichteten Zielmediums assoziiert sind, zu der die neue ausgewählte Modellkonfiguration des geschichteten Mediums (CMirrad) gehört,
  * dann Bestimmen mindestens einer besonderen Modulationsfrequenz (foptim) auf der Basis der genannten mehreren Knotenmodulationsfrequenzen, wobei die genannte besondere Modulationsfrequenz ein gewichteter Durchschnitt von mindestens zwei der genannten mehreren Knotenmodulationsfrequenzen ist und durch zwei Knotenmodulationsfrequenzen begrenzt wird,
  * Bestimmen eines neuen besonderen Strahlungsfalls für die neue ausgewählte Modellkonfiguration des geschichteten Zielmediums (CMirrad), wobei der besondere Strahlungsfall die mindestens eine besondere Modulationsfrequenz (foptim) umfasst,
  * und Senden des neuen besonderen Strahlungsfalls zu der Vorrichtung zur Steuerung der Strahlungsparameter der Lichtquelle (11a), so dass die Lichtquelle (11a) das geschichtete Zielmedium (2) gemäß dem Satz von Strahlungsparametern des genannten neuen besonderen Strahlungsfalls bestrahlt, die Detektionszelle (12) ein als Reaktion auf diese neue Bestrahlung erzeugtes akustisches oder thermisches Signal detektiert und der Prozessor des Anpassungsmoduls (14) den Parameter von Interesse auf der Basis des neuen detektierten akustischen oder thermischen Signals bestimmt;

- Bestimmen des gemessenen Wertes des Parameters von Interesse (Pmes) auf der Basis des letzten geschätzten Wertes des Parameters von Interesse (Pest).

10. Nichtinvasiver Sensor nach einem der Ansprüche 8 und 9, wobei mindestens zwei der Elemente aus der Lichtquelle (11a), der Vorrichtung zur Steuerung der Strahlungsparameter der Lichtquelle (11a), der Detektionszelle (12), die zum Detektieren eines akustischen oder thermischen Signals konfiguriert ist, dem Speicher, in dem ein Knotenfrequenzdiagramm gespeichert ist, und dem Anpassungsmodul (14) mechanisch voneinander unabhängig sind.

11. Computerprogramm mit Befehlen, die den nichtinvasiven Sensor (1) nach einem der Ansprüche 8 bis 10 zum Ausführen der Schritte des Verfahrens nach einem der Ansprüche 1 bis 7 veranlassen.


**Claims**

1. A method of measuring a parameter of interest in a stratified target environment (2) by means of a non-invasive sensor (1) based on photoacoustic detection or photothermal detection, comprising:

   a) a sensor is provided comprising :

   - a light source (11a),
   - a device for controlling a plurality of irradiation parameters of the light source (11a), the plurality of irradiation parameters comprising at least one frequency for modulating the intensity of the light source,
   - a detection cell configured to detect an acoustic or thermal signal (12),
   - a memory in which is stored a nodal frequency abacus comprising, for :

      * a plurality of groups (GMp) of model configurations of the stratified target environment, each comprising at least two model configurations (CMk) of the stratified target environment differing from one another only by the parameter of interest,
      * and a plurality of irradiation case groups (GIq), each comprising at least two irradiation cases (Ij) differing from one another only in a modulation frequency of a light source, each irradiation case (Ij) comprising a set of irradiation parameter values,

      a plurality of nodal multiplets, each nodal multiplet comprising characteristics common to all the elements of a given group (GMp) of model configurations of the stratified target environment and a plurality of associated nodal modulation frequencies, for which the acoustic or thermal signal detected by the detection cell in response to irradiation by the light source exhibits a correlation below a predetermined threshold with the parameter of interest ;
   - and an adaptation module (14) comprising a processor and exchanging information with the detection cell (12) and the light source irradiation parameter control device;

   b) the adaptation module (14) selects a model configuration of the initial stratified target environment (CMirrad) for irradiation;
   c) the processor of the adaptation module (14) :

   - determines in the nodal frequency abacus a plurality of nodal modulation frequencies (fnod) associated with a group (GMp) of model configurations of the stratified target environment to which the chosen model configuration of the stratified environment (CMirrad) belongs,
   - then determines at least one particular modulation frequency (foptim) on the basis of said plurality of nodal modulation frequencies, said particular modulation frequency being a weighted average of at least two of said plurality of nodal modulation frequencies and being bounded by two nodal modulation frequencies,
   - and determines a particular irradiation case for the chosen model configuration of the stratified target environment (CMirrad), the particular irradiation case comprising the at least one particular modulation frequency (foptim);

   d) the light source (11a) irradiates the stratified target environment (2) according to the set of irradiation parameters of said particular irradiation case;
   e) the detection cell (12) detects an acoustic or thermal signal generated in response to irradiation;
   f) the processor of the adaptation module (14) determines the parameter of interest on the basis of the acoustic or thermal signal detected;

2. Measurement method according to claim 1 wherein :

- the processor of the adaptation module (14) further implements an inverse modeling algorithm receiving as input an irradiation case (Ij) and an acoustic or thermal signal and providing as output a model configuration (CMk) of the stratified target environment and a value of the parameter of interest,
- step f) comprises the following steps:

f1) the adaptation module processor receives as input the acoustic or thermal signal detected by the detection cell (12) and the particular irradiation case used for irradiation, and returns as output from the inverse modeling algorithm a model configuration of the current stratified target environment (CMmes) and an estimated vamue oh the parameter of interest (Pest);
f2) the adaptation module processor (14) evaluates the chosen stratified target environment model configuration (CMirrad) by comparison with the current stratified target environment model configuration (CMmes), and, only if CMirrad is different from CMmes: g) the adaptation module (14) receives as input the model configuration of the current stratified target environment (CMmes) and returns as output a new model configuration of the chosen stratified target environment for irradiation (CMirrad), then c), d), e) and f1) are repeated;
f3) the value of the parameter of interest measured (Pmes) by the sensor is the last value of the parameter of interest estimated (Pest);

3. Measurement method according to claim 1, g) further comprising repeating f2) at the end of f1);

4. Measurement method according to one of claims 1 to 3 comprising beforehand :

I- by means of a processor and a database of model configurations comprising multiplets (model configuration (CMk) of the stratified target environment, irradiation case (Ij), parameter of interest) and an acoustic or thermal signal detected by the detection cell (12) associated with each of said multiplets, a nodal frequency abacus is generated for :

- a plurality of groups (GMp) of model configurations,
- and a plurality of groups (GIq) of irradiation cases,
the nodal frequency abacus comprises a plurality of nodal multiplets, each nodal multiplet comprising characteristics common to all the elements of a group (GMp) of model configurations of the stratified target environment and a plurality of associated nodal modulation frequencies,
and this nodal frequency abacus is stored in the memory of the non-invasive sensor (1);

5. Measurement method according to claim 4 comprising :
II- a processor learns at least one inverse modeling algorithm from the model configuration database, and the at least one inverse modeling algorithm is stored in the memory of the non-invasive sensor (1);

6. Measurement method according to any one of claims 4 to 5 in which at least one of the acoustic or thermal signals detected by the detection cell (12) associated with the multiplets stored in the model configuration database are simulated, i.e. generated by means of a computerized simulation device;

7. Measurement method according to any one of claims 1 to 6 wherein said particular irradiation case for the chosen model configuration determined by the adaptation module (14) in step c) further comprises at least a nodal modulation frequency;

8. A non-invasive sensor (1) based on photoacoustic or photothermal detection configured to measure a parameter of interest in a stratified target environment (2) comprising:

- a light source (11a),
- a device for controlling the irradiation parameters of the light source (1 1a),
- a detection cell (12) configured to detect an acoustic or thermal signal,
- a memory in which is stored a nodal frequency abacus comprising, for :
- a plurality of groups (GMp) of model configurations of the stratified target environment, each comprising at least two model configurations (CMk) of the stratified target environment differing from one another only by the parameter of interest,

- and a plurality of groups (GIq) of irradiation cases, each comprising at least two irradiation cases (Ij) differing from one another only in a modulation frequency of a light source, each irradiation case (Ij) comprising a set of irradiation parameter values,

A plurality of nodel multiplets, each nodal multiplet comprising characteristics common to all the elements of a given group (GMp) of model configurations of the stratified target environment and a plurality of associated nodal modulation frequencies, for which the acoustic or thermal signal detected by the detection cell in response to irradiation by the light source exhibits a correlation below a predetermined threshold with the parameter of interest,

the non-invasive sensor further comprising an adaptation module (14) comprising a processor and adapted to exchange information with the detection cell (12) and the light source irradiation parameter control device (11a), the adaptation module (14) being further configured to :

- select a model configuration of the initial stratified target environment (CMirrad),
- determine in the nodal frequency abacus a plurality of nodal modulation frequencies (fnod) associated with a group (GMp) of model configurations to which the selected stratified target environment model configuration (CMirrad) belongs,
- determine at least one particular modulation frequency (foptim) on the basis of said plurality of nodal modulation frequencies, said particular modulation frequency being a weighted average of at least two of said plurality of nodal modulation frequencies and being bounded by two nodal modulation frequencies;
- determine a particular irradiation case for the selected irradiation model configuration (CMirrad) comprising the at least one particular modulation frequency (foptim);
- transmit a particular irradiation case to the light source irradiation parameter control device,
- receive an acoustic or thermal signal detected by the detection cell (12),
- determine the value of the parameter of interest on the basis of the acoustic or thermal signal detected.

9. A non-invasive sensor according to claim 8 wherein the processor of the adaptation module is further adapted to :

- implementing an inverse modeling algorithm receiving as input an irradiation case (Ij) comprising a set of irradiation parameters and an acoustic or thermal signal and providing as output a model configuration (CMk) of the stratified target environment and a value of the parameter of interest;
- using the inverse modeling algorithm, determine a current model configuration of the stratified target environment (CMmes) and an estimated value of the parameter of interest (Pest) on the basis of a detected photoacoustic or photothermal signal received and a selected irradiation case;
- evaluate a chosen model irradiation configuration of the stratified target environment (CMirrad) by comparison with a current model configuration of the stratified target environment (CMmes);
- only if the model irradiation configuration of the chosen target stratified environment (CMirrad) and the model configuration of the current target stratified environment (CMmes) compared are different:

* determine a new model irradiation configuration of the selected stratified target environment (CMirrad) when it receives a model irradiation configuration of the current stratified target environment (CMmes),
* determine in the abacus of nodal frequencies a plurality of nodal modulation frequencies (fnod) associated with a group of model configuration (GMp) of the target stratified environment to which the new model configuration of the chosen stratified environment (CMirrad) belongs,
* then determine at least one particular modulation frequency (foptim) on the basis of said plurality of nodal modulation frequencies, said particular modulation frequency being a weighted average of at least two of said plurality of nodal modulation frequencies and being delimited by two nodal modulation frequencies,
* determine a new particular irradiation case for the new model configuration of the chosen target stratified environment (CMirrad), the particular irradiation case comprising the at least one particular modulation frequency (foptim),
* and transmit the new particular irradiation case to the device for controlling the irradiation parameters of the light source (11a) so that the light source (11a) irradiates the target stratified environment (2) following the set of irradiation parameters of said new particular irradiation case, that the detection cell (12) detects an acoustic or thermal signal generated in response to this new irradiation and the processor of the adaptation module (14) determines the parameter of interest based on the new acoustic or thermal signal detected;

- determine the measured parameter of interest value (Pmes) on the basis of the last estimated value of the parameter of interest (Pest);

10. Non-invasive sensor according to any one of claims 8 and 9 in which at least two of the devices among the light source (11a), the device for controlling the irradiation parameters of the light source (11a), the detection cell (12) configured to detect an acoustic or thermal signal, the memory in which an abacus of nodal frequencies is stored, and the adaptation module (14) are mechanically independent of each other;

11. A computer program comprising instructions which lead the non-invasive sensor (1) according to any one of claims 8 to 10 to perform the steps of the process according to any one of claims 1 to 7.

Fig. 1

Fig. 2

Fig. 3a

Fig. 3b

Fig. 4

## Fig. 5

**Initialisation** : choix de la configuration modèle initiale
CMirrad = CMk
$N_k, \{e_{i,k}, [H_2O]_{i,k}, ....\}$  $i \in [|1, N_k|]$     (sauf [Glc])

**Irradiation initiale** avec un cas d'irradiation comprenant au moins une source de lumière modulée à une fréquence de modulation particulière pour CMirrad = CMk

**Détection**

Amplitude et phase pour tous les couples $\{f_{mod,p} ; \lambda_p\}$   du cas d'irradiation choisi

**Irradiation ultérieure** avec au moins une source de lumière modulée en intensité à au moins une fréquence de modulation

**Résolution du problème inverse**
Détermination de la configuration modèle CMmes correspondant au signal photoacoustique ou photothermique

**Choix** d'une nouvelle configuration modèle
CMirrad = CMmes

**Validation** de la configuration modèle :
CMmes = CMirrad?

non

oui

[Glc] estimée

Fig. 6

Fig. 7

Fig. 8a

Erreur quadratique moyenne: 18.5 mg/dL

Fig. 8b

Erreur quadratique moyenne: 28.4 mg/dL

Fig. 8c

Erreur quadratique moyenne: 15.4 mg/dL

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **ROSENCWAIG, A. ; GERSHO, A.** Theory of the photoacoustic effect with solids. *Journal of Applied Physics,* 1976, vol. 47, 64
- **HU, H. ; WANG, X. ; XU, X.** Generalized theory of the photoacoustic effect in a multilayer material. *Journal of Applied Physics,* 1999, vol. 86, 3953-3958
- **C. A. BENNETT ; R. R. PATTY.** Thermal wave interferometry: a potential application of the photoacoustic effect. *Appl. Opt.,* 1982, vol. 21, 49-54
- **ANDREAS MANDELIS.** Theory of photothermal-wave diffraction and interférence in condensed media. *J. Opt. Soc. Am. A,* 1989, vol. 6, 298-308
- **ANDREAS MANDELIS ; KWAN F. LEUNG.** Photothermal-wave diffraction and interférence in condensed media: experimental evidence in aluminum. *J. Opt. Soc. Am. A,* 1991, vol. 8, 186-200
- **HU, HANPING et al.** Generalized theory of the photoacoustic effect in a multilayer material. *Journal of Applied Physics,* 1999, vol. 86, 3953-3958
- **CAO, J.** Interferential formulization and interprétation of the photoacoustic effect in multilayered cells. *Journal of Physics D: Applied Physics,* 2000, vol. 33 (3), 200
- **KOTTMANN, J. et al.** Glucose sensing in human epidermis using mid-infrared photoacoustic détection. *Biomedical optics express,* 2012, vol. 3 (4), 667-680